# EUROPÄISCHE PATENTSCHRIFT

(11) **EP 2 121 627 B1**
(45) Veröffentlichungstag und Bekanntmachung des Hinweises auf die Patenterteilung: **18.08.2010**
(21) Anmeldenummer: 08707339.1
(22) Anmeldetag: 28.01.2008
(51) Int. Cl.: C07D 235/18, C07D 401/04, A61K 31/4184, A61K 31/44, A61P 3/10

(54) **BENZIMIDAZOLDERIVATE**
BENZIMIDAZOLE DERIVATIVES
DÉRIVÉS DE BENZIMIDAZOLE

(30) Priorität: 21.02.2007 DE 102007008420
(43) Veröffentlichungstag der Anmeldung: 25.11.2009
(73) Patentinhaber: Merck Patent GmbH, 64293 Darmstadt (DE)
(72) Erfinder: BURGDORF, Lars, Thore, 60389 Frankfurt am Main (DE); CEZANNE, Bertram, 64546 Moerfelden-Walldorf (DE); KLEIN, Markus, 64291 Darmstadt (DE); GERICKE, Rolf, 64342 Seeheim-Jugenheim (DE); TSAKLAKIDIS, Christos, 69469 Weinheim (DE); MEDERSKI, Werner, 64673 Zwingenberg (DE); BEIER, Norbert, 64354 Reinheim (DE)
(86) Internationale Anmeldenummer: PCT/EP2008/000633
(87) Internationale Veröffentlichungsnummer: WO 2008/101586

(56) Entgegenhaltungen:
- EP-A- 1 721 904
- WO-A-2007/014894
- ANTHONY L. HANDLON: "Sodium glucose co-transporter 2 (SGLT2) inhibitors as potential antidiabetic agents" EXPERT OPINION ON THERAPEUTIC PATENTS, Bd. 15, Nr. 11, 2005, Seiten 1531-1540, XP002477436

## Beschreibung

Die Erfindung betrifft Verbindungen der Formel I 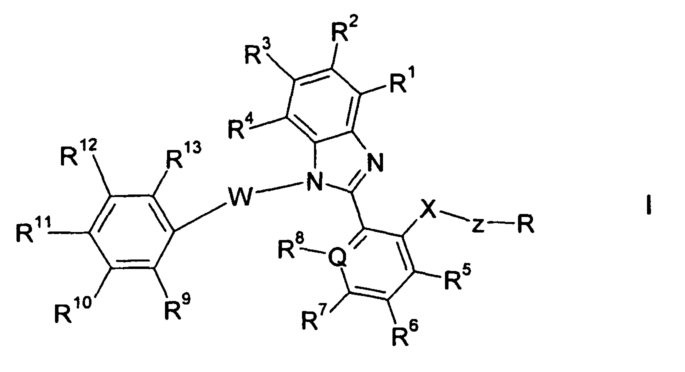 worin
- R¹, R², R³, R⁴: jeweils unabhängig voneinander H, A oder Hal,
- R⁵, R⁶, R⁷, R⁸: jeweils unabhängig voneinander H, A, Hal, OH oder OA, wobei R⁸ fehlt, falls Q = N,
- R⁹, R¹⁰, R¹¹, R¹², R¹³: jeweils unabhängig voneinander H, A oder Hal, wobei einer der Reste R⁹, R¹⁰, R¹¹, R¹² oder R¹³ ≠ H ist,
- R: CO-NR¹⁸R¹⁹ oder Het,
- Q: N oder C,
- X: O oder NR¹⁴R¹⁵,
- W: (CR¹⁴R¹⁵)ₘ, CO, NR¹⁴R¹⁵, S(O)ₙ oder fehlt,
- Z: (CR²⁰R²¹)ₘ,
- R¹⁴, R¹⁵: jeweils unabhängig voneinander H oder A,
- R¹⁶, R¹⁷: jeweils unabhängig voneinander H oder A,
- R¹⁸, R¹⁹: jeweils unabhängig voneinander H oder A,
- R²⁰: H oder A,
- R²¹: H, A oder CH₂OH,
- R¹⁸ und R²¹: zusammen auch (CR¹⁶R¹⁷)ₚ,
- Het: einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch Hal, A, OH, OA, NR¹⁴R¹⁵ und/oder =O (Carbonylsauerstoff) substituiert sein kann,
- A: unverzweigtes oder verzweigtes Alkyl mit 1-10 C- Atomen, worin 1-7 H-Atome durch F ersetzt sein können,
- Hal: F, Cl, Br oder I,
- m: 0, 1, 2, 3 oder 4,
- n: 0, 1 oder 2,
- p: 1, 2 oder 3,
bedeuten,
sowie ihre pharmazeutisch verwendbaren Solvate, Salze und Stereoisomere, einschließlich deren Mischungen in allen Verhältnissen.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Sie zeigen SGLT1 und SGLT2 (sodium dependent glucose co-transporter) inhibierende Eigenschaften und können daher zur Bekämpfung und Verhütung von Diabetes vom Typ 1 und Typ 2 eingesetzt werden.

Die Absorption von Glucose im Bürstensaum des Dünndarms und in den proximalen Nierentubuli gegen einen Konzentrationsgradienten erfolgt über epitheliale natriumabhängige Glucose-Cotransporter (SGLTs). Es wurden mindestens zwei größere Klassen von SGLTs beschrieben: SGLT1 (beispielsweise Lee W.S. et al. (1994) The high-affinity Na+/Glucose co-transporter: reevaluation of function and distribution of expression. J. Biol. Chem. 269, 12032-12039) und SGLT2 (beispielsweise Mackenzie B. et al. (1994) SAAT1 ist a low-affinity Na+/glucose cotransporter and not an amino acid transporter. J. Biol. Chem. 269, 22488-22491).
Es wird angenommen, dass SGLT1 für die Absorption von Glucose im Darm wichtig ist, wohingegen SGLT2 wahrscheinlich für die Reabsorption von frei filtrierter Glucose in der Niere hauptsächlich verantwortlich ist.

Die hauptsächliche Veränderung bei Diabetes mellitus ist Hyperglykämie. Dies ist nicht nur ein Symptom der Erkrankung, sondern auch ein potentieller pathogener Faktor, der zu multiplen chronischen diabetischen mikro- und makrovaskularen Komplikationen und einer Störung der Insulinsekretion und Empfindlichkeit führt (Klein R. (1995), Hyperglycemia and microvascular and macrovascular disease in diabetes, Diabetes Care 18, 258-268; Rossetti L. (1995), Glucose toxicity: the implications of hyperglycemia in the pathophysiology of diabetes mellitus, Clin. Invest. Med. 18, 255-260). Somit ist beim Diabetes-Patient die ausschließliche Regulation der Blut-Glucosespiegel innerhalb des normalen Bereichs ein wichtiges Therapieziel. Entsprechend ihrer beschriebenen Funktion führt eine Hemmung der SGLTs zu einer verringerten Absorption und gesteigerten Ausscheidung von Glucose, sowie zu einer anschließenden Abnahme der Blut-Glucosespiegel. Somit kann die Unterdrückung der SGLTs eine geeignete Alternative zur Behandlung von Diabetes sein.

In der Literatur sind mehrere Substanzklassen mit SGLT-Wirkung beschrieben. All diesen Strukturen diente als Leitbild der Naturstoff Phlorizin.
Aromatische Glycosidderivate kennt man aus WO 2004/052902 und WO 2004/052903. Propiophenonglycoside sind beschrieben in WO 0280936, WO 0280935, JP 2000080041 und EP 850948. Glucopyranoslyoxybenzylbenzole sind in WO 0244192, WO 0228872 und WO 0168660 beschrieben. Glucopyranosyloxy-pyrazole kennt man aus WO 0268440, WO 0268439, WO 0236602 und WO 0116147. O-Glycosidbenzamide sind in WO 0174835 und WO 0174834 offenbart. C-Arylglycoside sind in WO 0127128 und US 2002137903 beschrieben. Alle bekannten Strukturen enthalten als sehr wichtiges Strukturelement die Glucose. Ferner sind aus US 2002/132807 Diarylsulfid-Verbindungen zur Behandlung von Entzündungs- und Immun-Erkrankungen bekannt. In EP 0 953 357 A1 werden allgemein Glycosid-Verbindungen als renale Drug-Carrier und in WO 95/23780 4-Hydroxy-phenoxy-hetero-cycloalkyl-Verbindungen als Hautaufheller beschrieben.
Andere Indolizinderivate kennt man aus WO 2004/108722 und aus Bioorg. Med. Chem. Lett. 2006, 16, 3998ff.

Die erfindungsgemäßen Verbindungen weisen ein hohes Splitting in bezug auf die gewünschte Affinität von SGLT₂ zu SGLT₁ auf.

Die Verbindungen der Formel I zeichnen sich durch günstige Wirkungen auf den Glucosestoffwechsel aus, sie senken insbesondere den Blutzuckerspiegel und sind zur Behandlung von Typ 1 und Typ 2 Diabetes geeignet. Die Verbindungen können daher allein oder in Kombination mit weiteren Blutzucker-senkenden Wirkstoffen (Antidiabetika) eingesetzt werden.

Die Verbindungen der Formel I eignen sich weiterhin zur Prävention und Behandlung von diabetischen Spätschäden, wie z.B. Nephropathie, Retinopathie, Neuropathie sowie Syndrom X, Obesitas, Herzinfarkt, myocardialem Infarkt, peripheren arteriellen Verschlusskrankheiten, Thrombosen, Arteriosklerose, Entzündungen, Immunkrankheiten, Autoimmunkrankheiten, wie z.B. AIDS, Asthma, Osteoporose, Krebs, Psoriasis, Alzheimer, Schizophrenie und Infektionskrankheiten, bevorzugt ist die Behandlung von Typ 1 und Typ 2 Diabetes sowie zur Prävention und Behandlung 15 von diabetischen Spätschäden, Syndrom X und Obesitas.

Die Verbindungen der Formel I können als Arzneimittelwirkstoffe in der Human- und Veterinärmedizin eingesetzt werden, insbesondere zur Behandlung und Verhütung von Diabetes vom Typ 1 und Typ 2.

Gegenstand der Erfindung sind die Verbindungen der Formel I und ihre Salze sowie ein Verfahren zur Herstellung von Verbindungen der Formel I sowie ihrer pharmazeutisch verwendbaren Solvate, Salze und Stereoisomere, **dadurch gekennzeichnet, daß** man
a) eine Verbindung der Formel II 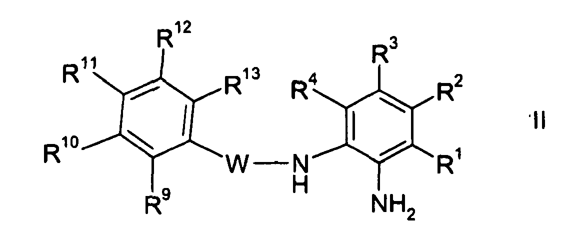 worin
   R¹, R², R³, R⁴, R⁹, R¹⁰, R¹¹, R¹², R¹³ und W die in Anspruch 1 angegebenen Bedeutungen haben,
   mit einer Verbindung der Formel III 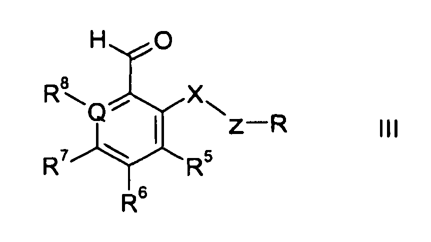 worin
   R⁵, R⁶, R⁷, R⁸, R, Q, W und Z die in Anspruch 1 angegebenen Bedeutungen haben,
   umsetzt,
   oder
b) eine Verbindung der Formel IV 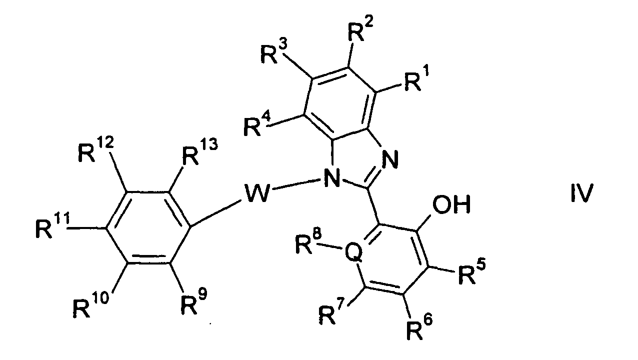 worin
   R¹, R², R³, R⁴, R⁵, R⁶, R⁷, R⁸, R⁹, R¹⁰, R¹¹, R¹², R¹³, Q und W die in Patentanspruch 1 angegebenen Bedeutungen haben,
   mit einer Verbindung der Formel V

   L-Z-R V

   worin Z und R die in Patentanspruch 1 angegebenen Bedeutungen haben und
   L Cl, Br, I oder eine freie oder reaktionsfähig funktionell abgewandelte OH-Gruppe bedeutet,
   umsetzt,
   und/oder
   eine Base oder Säure der Formel I in eines ihrer Salze umwandelt.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen als auch sogenannte Prodrug-Verbindungen.
Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in Int. J. Pharm. 115, 61-67 (1995) beschrieben ist.

Gegenstand der Erfindung sind auch Mischungen der erfindungsgemäßen Verbindungen der Formel I, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Für alle Reste, die mehrfach auftreten, gilt, daß deren Bedeutungen unabhängig voneinander sind.
Vor- und nachstehend haben die Reste bzw. Parameter R¹, R², R³, R⁴, R⁵, R⁶, R⁷, R⁸, R⁹, R¹⁰, R¹¹, R¹², R¹³, R¹⁸, R¹⁹, Q, R, W, X und Z die bei der Formel I angegebenen Bedeutungen, falls nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

R¹, R², R³, R⁴ bedeuten vorzugsweise, jeweils unabhängig voneinander H, CH₃, F oder Cl.
R⁵, R⁶, R⁷, R⁸ bedeuten vorzugsweise, jeweils unabhängig voneinander H, CH₃, Hal oder OCH₃,
wobei R⁸ fehlt, falls Q = N ist.
R⁹, R¹⁰, R¹¹, R¹² , R¹³ bedeuten vorzugsweise, jeweils unabhängig voneinander H, CH₃, F oder Cl, wobei einer der Reste R⁹, R¹⁰, R¹¹, R¹² oder R¹³ ≠ H ist.
R⁹, R¹³ bedeuten besonders bevorzugt, jeweils unabhängig voneinander CH₃, F oder Cl.
R¹¹ bedeutet besonders bevorzugt H oder CH₃,
R¹⁰, R¹² bedeuten besonders bevorzugt H.
X bedeutet vorzugsweise O.
W bedeutet vorzugsweise (CR¹⁴R¹⁵)ₘ oder CO.
Q bedeutet vorzugsweise C.
R¹⁴, R¹⁵, R¹⁶, R¹⁷, R¹⁸, R¹⁹ bedeuten vorzugsweise H.
R²⁰ bedeutet vorzugsweise H.
R²¹ bedeutet vorzugsweise H oder CH₂OH.

Het bedeutet, ungeachtet weiterer Substitutionen, z.B. 2- oder 3-Furyl, 2-oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, Indazolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnoliriyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl, 2,1,3-Benzoxadiazol-5-yl oder Dibenzofuranyl.
Die heterocyclischen Reste können auch teilweise oder vollständig hydriert sein.

Ungeachtet weiterer Substitutionen kann Het also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder-8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydro-benzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl, 2,3-Dihydro-2-oxo-furanyl, 3,4-Dihydro-2-oxo-1H-chinazolinyl, 2,3-Dihydro-benzoxazolyl, 2-Oxo-2,3-dihydro-benzoxazolyl, 2,3-Dihydro-benzimidazolyl, 1,3-Dihydroindol, 2-Oxo-1,3-dihydro-indol oder 2-Oxo-2,3-dihydro-benzimidazolyl.

Het bedeutet vorzugsweise einen ein- oder zweikernigen gesättigten, ungesättigten oder aromatischen Heterocyclus mit 1 bis 4 N-, O- und/oder S-Atomen, der ein- oder zweifach durch NR¹⁴R¹⁵ und/oder =O (Carbonylsauerstoff) substituiert sein kann.
Het bedeutet besonders bevorzugt unsubstituiertes oder ein- oder zweifach durch NR¹⁴R¹⁵ und/oder =O (Carbonylsauerstoff) substituiertes Piperidinyl, Piperazinyl, Pyrrolidinyl, Morpholinyl, Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Indolyl, Benzo[1,3]dioxolyl, Indazolyl oder Benzo[2,1,3]thiadiazolyl.

Hal bedeutet vorzugsweise F, Cl oder Br, aber auch I.
m bedeutet vorzugsweise 0, 1 oder 2.
p bedeutet vorzugsweise 1 oder 2.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis In ausgedrückt werden, die der Formel I entsprechen und worin die nicht näher bezeichneten Reste die bei der Formel I angegebene Bedeutung haben, worin jedoch

| | | | |
|---|---|---|---|
| in Ia | A | unverzweigtes oder verzweigtes Alkyl mit 1, 2, 3, 4, 5 oder 6 C-Atomen, worin 1-5 H-Atome durch F ersetzt sein können, | |
| | bedeutet; | | |
| in Ib | R¹, R², R³, R⁴ | | jeweils unabhängig voneinander H, CH₃, F |
| | | | oder Cl, |
| | bedeuten; | | |
| in Ic | R⁵, R⁶, R⁷, R⁸ | | jeweils unabhängig voneinander H, CH₃, Hal oder OCH₃, |
| | | | wobei R⁸ fehlt, falls Q = N, |
| | bedeuten; | | |
| in Id | R⁹, R¹⁰, R¹¹, | | |
| | R¹², R¹³ | | jeweils unabhängig voneinander H, CH₃, F oder Cl, wobei einer der Reste R⁹, R¹⁰, R¹¹, R¹² oder R¹³ ≠ H ist, |
| | bedeuten; | | |
| in Ie | R⁹, R¹³ | | jeweils unabhängig voneinander CH₃, F oder Cl, |
| | R¹¹ | | H oder CH₃, |
| | R¹⁰, R¹² | | H |
| | bedeuten; | | |
| in If | X | | O bedeutet; |
| in Ig | W | | (CR¹⁴R¹⁵)ₘ oder CO bedeutet; |
| in Ih | R¹⁴, R¹⁵ | | H bedeuten; |
| in Ii | R¹⁶, R¹⁷ | | H bedeuten; |
| in Ij | R¹⁸, R¹⁹ | | H bedeuten; |
| in Ik | R²⁰ | | H, |
| | R²¹ | | H oder CH₂OH, |
| | bedeuten; | | |
| in Il | Het | einen ein- oder zweikernigen gesättigten, ungesättigten oder aromatischen Heterocyclus mit 1 bis 4 N-, O- und/oder S-Atomen, der ein- oder zweifach durch NR¹⁴R¹⁵ und/oder =O (Carbonylsauerstoff) substituiert sein kann, | |
| | bedeutet; | | |
| in Im | Het | unsubstituiertes oder ein- oder zweifach durch NR¹⁴R¹⁵ und/oder =O (Carbonylsauerstoff) substituiertes Piperidinyl, Piperazinyl, Pyrrolidinyl, Morpholinyl, Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Indolyl, Benzo[1,3]dioxolyl, Indazolyl oder Benzo[2,1,3]thiadiazolyl, | |
| | bedeutet; | | |
| in In | A | | unverzweigtes oder verzweigtes Alkyl mit 1, 2, 3, 4, 5 oder 6 C-Atomen, worin 1-5 H-Atome durch F ersetzt sein können, |
| | Het | | unsubstituiertes oder ein- oder zweifach durch NR¹⁴R¹⁵ und/oder =O (Carbonylsauerstoff) substituiertes Piperidinyl, Piperazinyl, Pyrrolidinyl, Morpholinyl, Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Indolyl, Benzo[1,3]dioxolyl, Indazolyl oder Benzo[2,1,3]thiadiazolyl, |
| | R¹, R², R³, R⁴ | | jeweils unabhängig voneinander H, CH₃, F |
| | | | oder Cl, |
| | R⁵, R⁶ R⁷, R⁸ | | jeweils unabhängig voneinander H, CH₃, Hal |
| | | | oder OCH₃, |
| | | | wobei R⁸ fehlt, falls Q = N, |
| | R⁹, R¹³ | | jeweils unabhängig voneinander CH₃, F oder Cl, |
| | R | | CO-NR¹⁸R¹⁹ oder Het, |
| | R¹¹ | | H oder CH₃, |
| | R¹⁰, R¹² | | H, |
| | Q | | N oder C, |
| | X | | O, |
| | W | | (CR¹⁴R¹⁵)ₘ oder CO, |
| | Z | | (CR²⁰R²¹)ₘ, |
| | R¹⁴, R¹⁵ | | H, |
| | R¹⁶, R¹⁷ | | H, |
| | R¹⁸, R¹⁹ | | H, |
| | R²⁰ | | H, |
| | R²¹ | | H oder CH₂OH, |
| | R¹⁸ und R²¹ | | zusammen auch (CR¹⁶R¹⁷)ₚ, |
| | m | | 0,1 oder 2, |
| | p | | 1 oder 2, |

bedeuten;
sowie ihre pharmazeutisch verwendbaren Solvate, Salze und Stereoisomere, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Ausgangsverbindungen der Formeln II,III und IV sind in der Regel bekannt. Sind sie neu, so können sie aber nach an sich bekannten Methoden hergestellt werden.

Verbindungen der Formel I können vorzugsweise erhalten werden, indem man Verbindungen der Formel II mit Verbindungen der Formel III umsetzt.

Die Umsetzung erfolgt in der Regel in einem inerten Lösungsmittel, in Gegenwart eines geeigneten Oxidationsmittels, wie z.B. Natriumdisulfit oder Sauerstoff.

Die Reaktionszeit liegt je nach den angewendeten Bedingungen zwischen einigen Minuten und 14 Tagen, die Reaktionstemperatur zwischen etwa 0° und 150°, normalerweise zwischen 30° und 140°, besonders bevorzugt zwischen 60° und 120°C.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel; besonders bevorzugt ist N-Methylpyrrolidon (NMP).

Verbindungen der Formel I können vorzugsweise weiterhin erhalten werden, indem man Verbindungen der Formel IV mit Verbindungen der Formel V umsetzt.

Die Umsetzung erfolgt in der Regel in einem inerten Lösungsmittel, in Gegenwart eines säurebindenden Mittels vorzugsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums. Auch der Zusatz einer organischen Base wie Triethylamin, Dimethylanilin, Pyridin oder Chinolin oder eines Überschusses der Phenolkomponente der Formel IV bzw. des Alkylierungsderivates der Formel V kann günstig sein. Die Reaktionszeit liegt je nach den angewendeten Bedingungen zwischen einigen Minuten und 14 Tagen, die Reaktionstemperatur zwischen etwa 0° und 150°, normalerweise zwischen 10° und 130°, besonders bevorzugt zwischen 20 und 80°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel. Besonders bevorzugt ist Acetonitril.

In den Verbindungen der Formel V bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy). L bedeutet besonders bevorzugt Br.

### Pharmazeutische Salze und andere Formen

Die genannten Verbindungen der Formel I lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der Verbindungen der Formel I Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der Formel I der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche Verbindungen der Formel I hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von sauren Verbindungen der Formel I werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine Verbindung der Formel I mehr als eine Gruppe, die solche pharmazeutisch unbedenklichen Salze bilden kann, so umfaßt die Formel I auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/ Acetonitril z.B. im Verhältnis 82:15:3.

Gegenstand der Erfindung ist ferner die Verwendung der Verbindungen der Formel I und/oder ihrer physiologisch unbedenklichen Salze zur Herstellung eines Arzneimittels (pharmazeutische Zubereitung), insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine Verbindung der Formel I und/oder ihre pharmazeutisch verwendbaren Solvate, Salze und Stereoisomere, einschließlich deren Mischungen in allen Verhältnissen, sowie gegebenenfalls Träger- und/oder Hilfsstoffe.

Diese Zubereitungen können als Arzneimittel in der Human- oder Veterinärmedizin verwendet werden.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nicht-toxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder tröckenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die Wirkstoffe können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindungen enthält. Sirupe lassen sich herstellen, indem die Verbindungen in einer wäßrigen Lösung mit geeignetem Geschmack gelöst werden, während Elixiere unter Verwendung eines nichttoxischen alkoholischen Vehikels hergestellt werden. Suspensionen können durch Dispersion der Verbindungen in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I sowie Salze, Solvate und physiologisch funktionelle Derivate davon sowie die anderen Wirkstoffe lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I sowie die Salze, Solvate und physiologisch funktionellen Derivate davon sowie die anderen Wirkstoffe können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I sowie des anderen Wirkstoffs hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer Verbindung im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der Verbindung per se bestimmt werden.

Gegenstand der Erfindung ist ferner die Verwendung von Verbindungen der Formel I, in Kombination mit mindestens einem weiteren Arzneimittelwirkstoff, vorzugsweise zur Behandlung des Typ 1 und Typ 2 Diabetes, insbesondere zur Blutzuckersenkung.

Als weitere Wirkstoffe für die Kombinationspräparate sind geeignet:

Alle Antidiabetika, die in der Roten Liste 2001, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart. Antidiabetika umfassen Insulin und Insulinderivate, wie z.B. Lantus^{®} (siehe www.lantus.com) oder HMR 1964, schnell wirkende Insuline (siehe US 6,221,633), GLP-1-Derivate wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulphonylharnstoffe, Biguanidine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glycogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT- 501, GI 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, AVE 0897 oder wie in WO 00/64888, WO 00/64876, WO 03/20269 beschrieben, verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide, BMS-201038, R-103757, verabreicht. Bei einer Ausführungsform der Erfindung werden die Verbindungen der Formel I in Kombination mit Gallensäureresorptionsinhibitor (siehe z.B. US 6,245,744 oder US 6,221, 897), wie z.B. HMR 1741, verabreicht.

Bei einer Ausführungsform der Erfindung werden die Verbindungen der Formel I in Kombination mit einem CETP-Inhibitor, wie z.B. JTT-705, verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht. Bei einer Ausführungsform der Erfindung werden die Verbindungen der Formel I in Kombination mit einem Lipoprotein(a) antagonist, wie z.B. Cl-1027 oder Nicotinsäure, verabreicht. Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.

Bei einer Ausführungsform werden die Verbindungen der Formel I in Kombination mit einem Sulphonylharnstoff, wie z.B. Tolbutamid, Glibenclamid, Glipizid oder Glimepirid verabreicht.

Bei einer Ausführungsform werden die Verbindungen der Formel I in Kombination mit einem Biguanid, wie z.B. Metformin, verabreicht.

Bei einer anderen Ausführungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform werden die Verbindungen der Formel I in Kombination mit einem α-Glukosidase-Inhibitor, wie z.B. Miglitol oder Acarbose, verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamineregulated transcript influences energy metabolism, anxiety and gastric emptying in mice" Asakawa, A, et al., M.:Hormone and Metabolic Research (2001), 33(9), 554-558), NPY-Antagonisten z.B. Naphthalin-1-sulfonsäure {4-[(4- amino-quinazolin-2-ylamino)-methyl]-cyclohexylmethyt}- amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]amid; (WO 01/91752)), Orexin-Antagonisten (z.B. 1-(2-Methyl- benzoxazol-6-yl)-3-[1,5]naphthyridin-4-yl-harnstoff; hydrochloride (SB-334867-A)), H3-Agonisten (3-Cyclohexyl-1-(4,4-dimethyl-1,4,6,7-tetrahydro-imidazo[4,5-c]pyridin-5-yl)-propan-1-on Oxalsäuresalz (WO 00/ 63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B.1-(4-Chloro-3-methanesulfonylmethyl-phenyl)-2-[2-(2,3-dimethyl-1 H-indol-6-yloxy)ethylamino]-ethanol; hydrochloride (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexylethyl)-thiazol-2-ylcarbamoyl]-5,7-dimethyl-indol-1-yl}-essigsäure Trifluoressigsäuresalz (WO 99/15525)); Serotonin-Wiederaufnahme-Inhibitoren (z.B. Dexfenfluramine), gemischte Serotonin- und noradrenerge Verbindungen (z.B. WO 10 00/71549), 5HT-Agonisten z.B. 1-(3-Ethyl-benzofuran-7-yl)-piperazin Oxalsäuresalz (WO 01/09111), Bombesin-Agonisten, Galanin- Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isochinolin-2-carbonsäure-tert-butylester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity, Drugs of the Future (2001), 26(9), 873-881), DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin; siehe z.B. "Perspectives in the therapeutic use of leptin", Salvador, Javier; Gomez Ambrosi, Javier; Fruhbeck, Gema, Expert Opinion on Pharmacotherapy (2001), 2(10), 1615-1622.

Bei einer Ausführungsform ist der weitere Wirkstoff Dexamphatamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Orlistat.

Bei einer Ausführungsform ist der weitere Wirkstoff Mazindol oder Phentermin.

Bei einer Ausführungsform werden die Verbindungen der Formel I in Kombination mit Ballaststoffen, vorzugsweise unlöslichen Ballaststoffen (siehe z.B. Carob/ Carornax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties & Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, daß jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

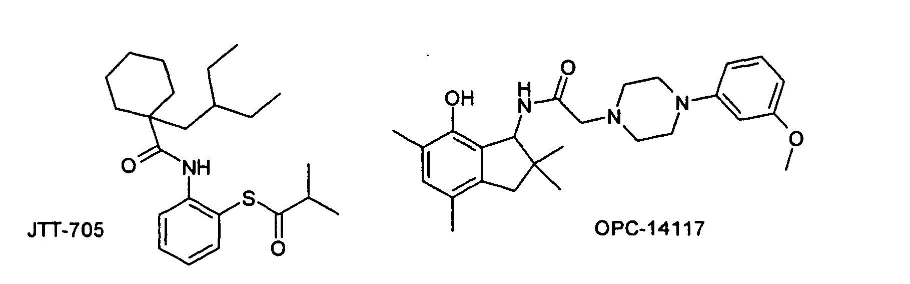

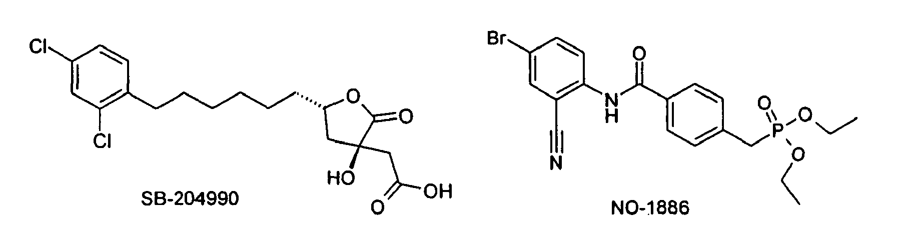

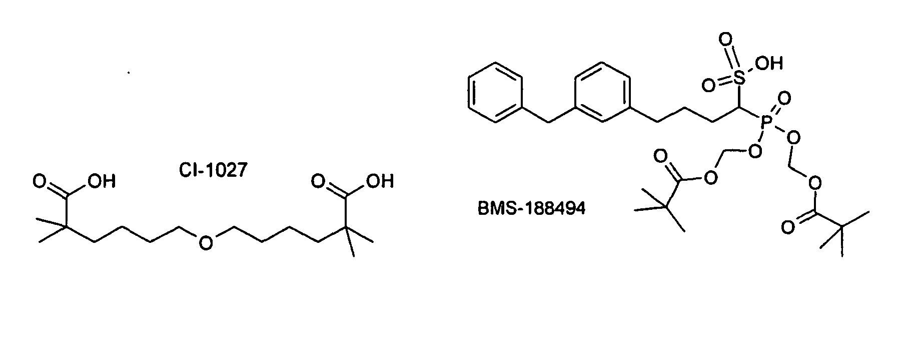

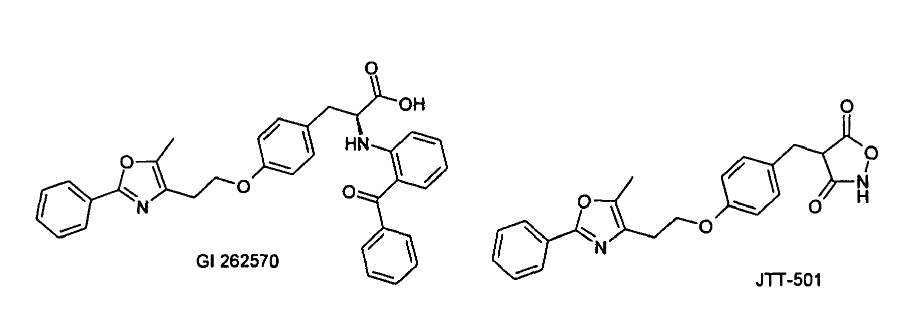

Gegenstand der Erfindung ist auch ein Set (Kit), bestehend aus getrennten Packungen von
(a) einer wirksamen Menge an einer Verbindung der Formel I und/oder ihrer pharmazeutisch verwendbaren Derivate, Solvate, Salze und Stereoisomere, einschließlich deren Mischungen in allen Verhältnissen,
   und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Solvate und Stereoisomere, einschließlich deren Mischungen in allen Verhältnissen,
und einer wirksamen Menge eines weiteren Arzneimittelwirkstoffs gelöst oder in lyophilisierter Form vorliegt.

Die Verbindungen können auf ihre SGLT-Hemmeigenschaften mittels BHK-Zellen getestet werden, die SGLT1 und SGLT2 exprimieren. Die Herstellung der Zellen und die Untersuchung kann wie nachstehend beschrieben durchgeführt werden.

### Konstruktion und Expression von SGLT1 in BHK-Zellen

Zur Konstruktion des SGLT1-Expressionsvektors (KL225) wurde das SLC5A1-Gen (homolog zu NM_000343) aus einer cDNA-Bank mittels Standard-PCR-Technologie amplifiziert und über NheI/XhoI-Stellen in den pcDNA3.1-Expressionsvektor (Invitrogen) kloniert, welcher Neomycin als Selektionsmarker enthielt. Bei diesem Vektor verwendet die Transkription den Enhancer/Promotor des Cytomegalievirus beim Menschen.

Der fertige Vektor KL225 wurde zusammen mit einem zusätzlichen Vektor, der ein Dihydrofolatreduktase-Gen als Selektionsmarker enthielt, in Zellen eingebracht. Die Transfektion in BHK21-Zellen (ATCC CCL-10), gezüchtet in DMEM-Medium (GIBCO/ BRL), angereichert mit 10% fötalem Kälberserum (FCS) und 20 mM Glutamin, erfolgte mit Calciumphosphat-Transfektionen gemäß Graham, F.L. und van der Ebb, A.J. (1973), Virology 52: 456 mit 5 - 20 µg ungeschnittenen Plasmiden für 10⁷ Zellen. Stabile Transfektanten wurden in Medium selektiert, das 1 mg/ml G418 (GIBCO/BRL) und 20 - 5000 nM Methotrexat als Endkonzentration enthielt, wobei nur Zellen, die das Neomycin-Gen exprimierten und das dhfr-Gen überexprimierten, wachsen konnten. Nach 2 - 3 Wochen Wachstum wurden die Zellen kloniert (0,5 Zellen/Vertiefung) und die Klone in Radioaktivitäts-Aufnahme-Tests auf SGLT-Expression untersucht.

### Konstruktion und Expression von SGLT2 in BHK-Zellen

Zur Konstruktion des SGLT2-Expressionsvektors (KL224) wurde das SLC5A2-Gen (homolog zu NM_003041) aus einer cDNA-Bank mittels Standard-PCR-Technologie amplifiziert und über NheI/XhoI-Stellen in PCIneo Expressionsvektor (Promega) kloniert, der Neomycin als Selektionsmarker enthielt. In diesem Vektor verwendet die Transkription den Enhancer/Promotor des Cytomegalie-Virus beim Menschen und das Polyadenylierungssignal von SV40.
Der fertige Vektor KL224 wurde zusammen mit einem zusätzlichen Vektor, der ein Dihydrofolatreduktase-Gen als Selektionsmarker enthielt, in Zellen eingebracht. Die Transfektion in BHK21-Zellen (ATCC CCL-10), gezüchtet in DMEM-Medium (GIBCO/ BRL), angereichert mit 10% fötalem Kälberserum (FCS) und 20 mM Glutamin, erfolgte mit Calciumphosphat-Transfektionen gemäß Graham, F.L. und van der Ebb, A.J. (1973), Virology 52: 456 mit 5 - 20 µg ungeschnittenen Plasmiden für 10⁷ Zellen. Stabile Transfektanten wurden in Medium selektiert, das 1 mg/ml G418 (GIBCO/BRL) und 20 - 5000 nM Methotrexat als Endkonzentration enthielt, wobei nur Zellen, die das Neomycin-Gen exprimierten und das dhfr-Gen überexprimierten, wachsen konnten. Nach 2 - 3 Wochen Wachstum wurden die Zellen kloniert (0,5 Zellen/Vertiefung) und die Klone in Radioaktivitäts-Aufnahme-Tests auf SGLT-Expression untersucht.

### Verfahren zur Messung der SGLT1/2-Aktivität

Prinzipiell wurde die Aufnahme von ¹⁴C-α-Methyl-D-glucopyranosid (AMG) beispielsweise in Xenopus-Oozyten, denen die entsprechende cRNA injiziert wurde, beschrieben (beispielsweise Wen-Sen Lee et al. (1994), J. Biol. Chem. 269, 12032-12039; Guofeng You et al. (1995), J. Biol. Chem. 270, 29365-29371).

Ein in 96 Vertiefungen durchgeführter Test auf Zellbasis wurde entwickelt und an die HTS-Anforderungen angepasst:
BHK-Zellen (transfiziert mit SGLT1 oder SGLT2) wurden in Mikrotiterplatten mit 96 Vertiefungen (Cultureplates, Perkin Elmer) überimpft. Nach mindestens 24 Std. wurde das Medium enternt und die Zellschicht wurde mit Testpuffer (140 mM NaCl, 2 mM KCI, 1 mM CaCl₂, 1 mM MgCl₂, 10 mM HEPES, 5 mM Tris, mit 1 M KOH auf pH-Wert 7,4 eingestellt) gewaschen. Nach Zugabe von 40 µl Testpuffer 50 µl AMG (50 µM für SGLT1 bzw. 2 mM für SGLT2) in Gegenwart oder Abwesenheit von Verbindungen wurden die Zellen in einem Gesamtvolumen von 100 µl bei 37°C für 90 min. inkubiert. Der Überstand wurde abgesaugt und verworfen. Die Zellen wurden gewaschen und durch Zugabe von 50 µl Wasser lysiert. Nach 10 min bei Raumtemperatur wurden 200 µl Micrsoscint 40 (Perkin Elmer) hinzu gefügt. Die Radioaktivität wurde in einem Topcount Mikroplatten-Szintillationszähler (Perkin Elmer) gezählt. Die unspezifische Aufnahme wurde in natriumfreiem Testpuffer (266 mM Saccharose, 2 mM KCI, 1 mM CaCl₂, 1 mM MgCl₂, 10 mM HEPES, 5 mM Tris, mit 1 M KOH auf pH-Wert 7,4 eingestellt) bestimmt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
Massenspektrometrie (MS): EI (Elektronenstoß-Ionisation) M⁺
FAB (Fast Atom Bombardment) (M+H)⁺

ESI (Electrospray lonization) (M+H)⁺ (wenn nichts anderes angegeben)

### LC-MS- und HPLC-Bedingungen

Die in den nachfolgenden Beispielen angegebenen M+H+-Daten sind die Meßergebnisse der LC-MS-Messungen:
Hewlett Packard System der HP 1100 Serie mit den folgenden Merkmalen: Ionenquelle: Elektrospray (positive mode); Scan: 100-1000 m/z; Fragmentier-Spannung: 60 V; Gas-Temperatur: 300°C, DAD: 220 nm.
Flussrate: 2.4 ml/Min. Der verwendete Splitter reduzierte nach dem DAD die Flussrate für das MS auf 0,75ml/Min.
Säule: Chromolith SpeedROD RP-18e 50-4.6
Lösungsmittel: LiChrosolv-Qualität der Fa. Merck KGaA
Lösungsmittel A: H2O (0.01% TFA)
Lösungsmittel B: ACN (0.008% TFA)

Die in den nachfolgenden Beispielen angegebenen Retentionszeiten Rt [min] sind die Meßergebnisse der LC-MS-Messungen.

### Beispiel 1

Die Herstellung von 2-{2-[1-(2,6-Dimethyl-benzyl)-6-fluor-1*H*-benzimidazol-2-yl]-5-methoxy-3-methyl-phenoxyfacetamid ("A1") erfolgt analog nachstehendem Schema: 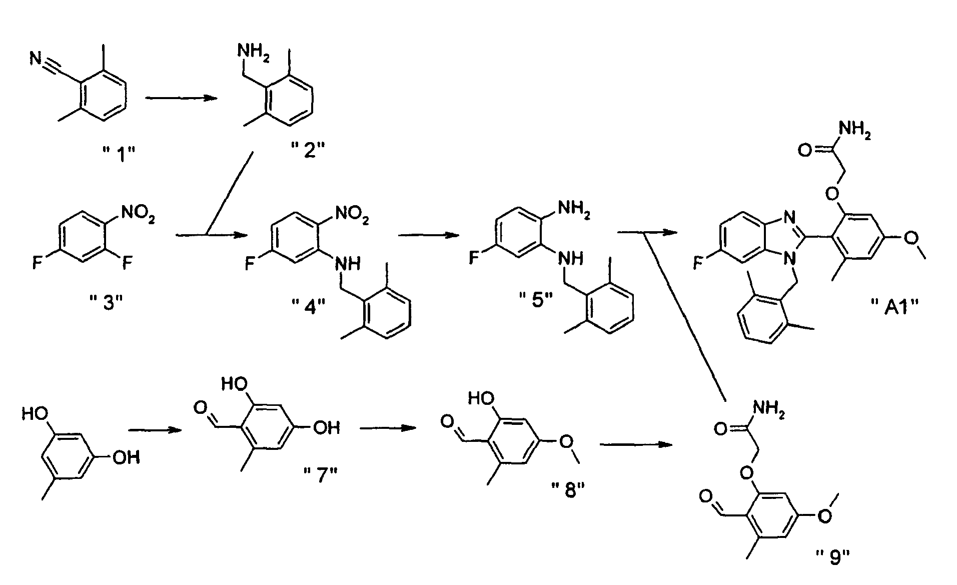
1.1 2,6-Dimethylbenzonitril ("1") wird in Methanol/NH₃ (w=10%) gelöst und über Raney-Nickel und Wasserstoff 16 Stunden bei 5 bar und 50 °C hydriert. Die Reaktionslösung wird über Celite filtriert und das Lösungsmittel am Vakuum entfernt. So wird das Produkt "2" in quantitativer Ausbeute als Öl erhalten; LC-MS: 1.307 min, m/z 136.5 (M+H⁺).
1.2 2,4-Difluor-1-nitrobenzol ("3", 1 eq) wird in DMF gelöst und mit einer Lösung aus "2" (1 eq) in DMF versetzt. Nach Zugabe von N-Ethyldiisopropylamin (1 eq) wird die Reaktion für 18 Stunden auf 85 °C erhitzt. Nach Abkühlen wird das Lösungsmittel am Vakuum entfernt, der Rückstand mit Diethylether aufgenommen und drei Mal mit Wasser gewaschen. Die organische Phase wird über Natriumsulfat getrocknet und das Lösungsmittel am Vakuum entfernt. So wird das Rohprodukt "4" mit einer Ausbeute von 64 % isoliert, welches ohne weitere Aufreinigung in der nächsten Stufe eingesetzt wird;. LC-MS: 2.496 min; m/z 275.15 (M+H⁺); 297.15 (M+Na⁺).
1.3 Nitroamin "4" (1 eq) wird in Eisessig suspendiert und Zink (grob gepulvert, 5.9 eq) hinzugefügt. Die Suspension wird innerhalb von 30 min zum Rückfluss für 3 Stunden erhitzt. Nach Abkühlen auf Raumtemperatur wird mit Wasser verdünnt und mit wässriger NaOH (32 %) auf pH 14 gestellt und vier Mal mit Dichlormethan extrahiert. Die vereinigten organischen Extrakte werden zwei Mal mit gesättigter NaCl Lösung gewaschen, über Natriumsulfat getrocknet und das Lösungsmittel am Vakuum entfernt. Das Rohprodukt wird säulenchromatographisch an Normalphase-Kieselgel gereinigt. So wird das Produkt "5" als Pulver mit einer Ausbeute von 39 % isoliert; LC-MS 1.591 min, m/z 245.15 (M+H⁺).
1.4 DMF (5 eq) wird auf <10 °C gekühlt und vorsichtig Phosphorylchlorid (1.3 eq) zugetropft. Anschliessend wird 3,5-Dihydroxytoluol (1 eq) in DMF (3.4 eq) vorsichtig bei < 10 °C zugetropft und über Nacht auf Raumtemperatur erwärmt. Die Suspension wird abgekühlt und mit Eis versetzt. Anschliessend wird mit konzentrierter NaOH auf pH 9-14 eingestellt und auf 90 °C erhitzt. Der pH wird dabei ständig mit konz. NaOH auf pH 9-14 neu eingestellt, solange bis sich der pH stabilisiert hat. Anschliessend wird auf 10 °C gekühlt und mit konzentrierter Salzsäure auf pH 1-3 gestellt. Die Suspension wird über Nacht abgekühlt, abgesaugt und mit Eiswasser gewaschen. Die Kristalle werden im Vakuum getrocknet. So wird das Produkt "7" als orangegelbes Pulver mit einer Ausbeute von 63 % isoliert; F. 181-183°; LC-MS: 0.974 min, m/z: 153.1 (M+H⁺).
1.5 Aldehyd "7" (1 eq) wird in Aceton gelöst, mit lodmethan (1.2 eq) und Kaliumcarbonat (2.2 eq) versetzt und 5 Stunden unter Rückfluss gekocht. Das Lösungsmittel wird am Vakuum entfernt und das Produkt mittels Säulenchromatographie an Normalphase-Kieselgel gereinigt. So wird das Produkt "8" mit einer Ausbeute von 68 % erhalten; LC-MS: 1.538 min, m/z 167.1 (MH⁺).
1.6 Aldehyd "8" (1 eq) wird in Acetonitril gelöst, mit Kaliumcarbonat (3 eq) und 2-lodacetamid (1.2 eq) versetzt. Die Lösung wird innerhalb von 20 Minuten zum Rückfluss erhitzt und 4 Stunden weiter gerührt. Nach Filtration kristallisiert die Substanz innerhalb von 2 Tagen aus. Die Kristalle werden abfiltriert, mit Acetonitril gewaschen und im Vakuum bei 40 °C getrocknet. So wird das Produkt "9" mit einer Ausbeute von 74 % isoliert; F. 173-174°; LC-MS: 0.975 min; m/z 224.15 (M+H⁺), 246.15 (M+Na⁺).
1.7 Diamin "5" (1 eq) und Aldehyd "9" (1.1 eq) werden in NMP gelöst und mit Natriumdisulfit (1.1 eq) versetzt. Die Lösung wird dann 4 Stunden bei 115 °C gerührt. Nach Abkühlen wird die Lösung in Wasser eingerührt und der entstandene Niederschlag abfiltriert und bei 40 °C getrocknet. Das Rohprodukt wird säulenchromatographisch an Normalphase-Kieselgel gereinigt. So wird das Produkt "A1" als farbloses Pulver mit einer Ausbeute von 74 % erhalten; LC-MS: 1.921 min; 448.15 m/z M+H⁺.

¹H-NMR (DMSO-d₆) δ [ppm] 7.79 (*dd*, 1H; *J*=8.9 Hz, *J*=8.9 Hz), 7.30-7.25 (*m*, 2H), 7.19 (*s*, 1H), 7.14 (*t*, 1H; *J*=7.5 Hz), 7.06 (*d*, 1H; *J*=8.0 Hz), 6.98 (*d*, 2H; 7.5 Hz), 6.58 (*d*, 1H; *J*=1.9 Hz), 6.40 (*d*, 1H; *J*=1.9 Hz), 5.59 (*d*, 1H; *J*=15.8 Hz), 5.36 (*d*, 1H; *J*=15.8 Hz), 4.41 (*d*, 1H; *J*=14.7 Hz), 4.36 (*d*, 1H; *J*=14.7 Hz), 3.81 (*s*, 3H), 1.99 (*s*, 6H), 1.98 (*s*, 3H).

### Beispiel 2

Die Herstellung von 2-{2-[1-(2,6-Dimethyl-benzyl)-1*H*-benzimidazol-2-yl]-phenoxy}-acetamid ("A2") erfolgt wie nachstehend angegeben:

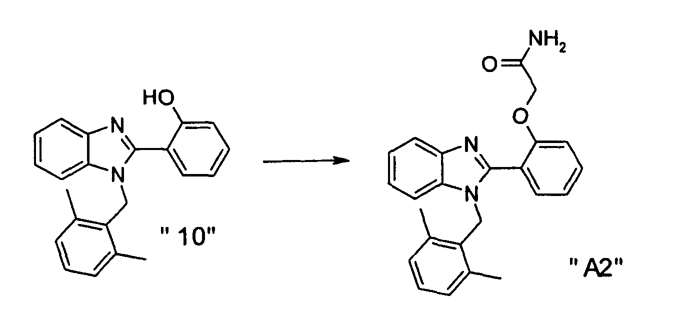

Die Herstellung des Alkohols "10" wird analog des vorher aufgeführten Beispiels 1 durchgeführt.

Alkohol "10" (1 eq) wird in Acetonitril gelöst, mit Kaliumcarbonat (3 eq) und 2-Bromacetamid (1.1 eq) versetzt. Die Lösung wird fünf Stunden bei Raumtemperatur gerührt und dann 22 Stunden zum Rückfluss erhitzt und vier Tage bei Raumtemperatur stehen gelassen. Nach Filtration wird das Lösungsmittel am Vakuum entfernt. Das Produkt "A2" wird nach säulenchromatographischer Reinigung an normal-phasen Kieselgel als Pulver mit einer Ausbeute von 8 % erhalten; LC-MS: 1.787 min; m/z 86.2 (M+H⁺).

¹H-NMR (DMSO-dₑ): δ [ppm] 7.81 (*d*, 1H; *J*=8.4 Hz), 7.71-7.70 (*m*, 1H), 7.67-7.64 (*m*, 1H), 7.54 (*s*, 1H), 7.45 (*t*, 1H; *J*=6.6 Hz), 7.41 (*s*, 1H), 7.33 (*t*, 1H; *J*=6.6 Hz), 7.22 (*t*, 1H; *J*=7.8 Hz), 7.14-7.11 (*m*, 3H), 6.98 (*d*, 2H; 8.4 Hz), 5.59 (*s*, 2H), 4.56 (*s*, 2H), 2.02 (*s*, 6H).

Analog erhält man die nachstehenden Verbindungen

| Nr. | Name und/oder Struktur | LC-MS Rt[min]; m/z [M+H]⁺ |
|---|---|---|
| "A3" | 2-{2-[1-(2,6-Dimethyl-benzyl)-1*H*-benzimidazol- | 1.412; |
| | 2-yl]-3-fluor-phenoxy}-acetamid | 404.8 |
| | 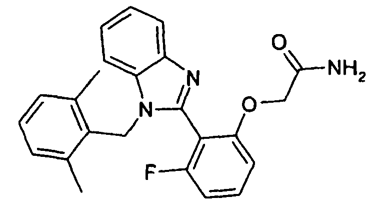 | |
| ¹H-NMR (DMSO-d₆): δ [ppm] 7.67 (*d*, 1H; *J*=8.0), 7.56-7.52 (*m*, 1H), 7.40 (*s*, 2H), 7.20 (*t*, 1H; *J*=8.0), 7.13-7.08 (*m*, 2H), 6.98-6.91 (*m*, 5H), 5.49 (*d*, 1H; J=16.2 Hz), 5.30 (*d*, 1H; *J*=16.2), 4.57 (*d*, 1H; *J*=15.9 Hz), 4.48 (*d*, 1H; *J*=15.9 Hz), 2.01 (*s*, 6H) | | |
| "A4" | 2-{2-[1-(2,6-Dimethyl-benzyl)-6-fluor-1*H*- | 1.880; |
| | benzimidazol-2-yl]-phenoxy}-acetamid 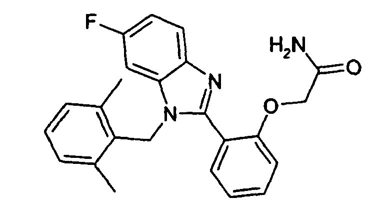 | 404.1 |
| ¹H-NMR (DMSO-d₆): δ [ppm] 7.72 (*dd*, 1H; *J*=4.9 Hz, *J*=8.6 Hz), 7.59-7.56 (*m*, 2H), 7.50 (*s*, 1H), 7.36 (*s*, 1H), 7.18-7.08 (*m*, 4H), 6.98 (*d*, 2H; *J*=7.6 Hz), 6.63 (*d*, 1H; *J*=9.6 Hz), 5.47 (*s*, 2H), 4.54 (*s*, 2H), 2.01 (*s*, 6H) | | |
| "A5" | 2-{2-[1-(2,6-Dimethyl-benzyl)-5,6-difluor-1*H*- | 1.731; |
| | benzimidazol-2-yl]-phenoxy}-acetamid | 422.1 |
| ¹H-NMR (DMSO-d₆): δ [ppm] 7.70 (*dd*, 1H; *J*=7.6 Hz, *J*=10.6 Hz), 7.56-7.48 (*m*, 3H), 7.34 (*s*, 1H), 7.15-7.07 (*m*, 3H), 6.99 (*d*, 2H; *J*=7.6 Hz), 6.67 (*dd*, 1H; *J*=7.4 Hz, *J*=11.1 Hz), 5.42 (*s*, 2H), 4.55 (*s*, 2H), 1.99 (*s*, 6H) | | |
| "A6" | 2-{2-[6-Fluor-1-(2,4,6-trimethyl-benzyl)-1*H*- | 1.593; |
| | benzimidazol-2-yl]-phenoxy}-acetamid | 418.1 |
| ¹H-NMR (DMSO-d₆): δ [ppm] 7.74 (*dd*, 1H; *J*=4.7 Hz, *J*=8.9 Hz), 7.62-7.56 (*m*, 2H), 7.51 (*s*, 1H), 7.38 (*s*, 1H), 7.18 (*t*, 2H; *J*=7.5 Hz), 7.12 (*d*, 1H; *J*=8.8 Hz), 6.82 (*s*, 2H), 6.66 (*d*, 1H; *J*=8.8 Hz), 5.43 (*s*, 2H), 4.56 (*s*, 2H), 2.21 (*s*, 3H), 1.98 (*s*, 6H) | | |
| "A7" | 2-{2-[1-(2-Methyl-benzyl)-1*H*-benzimidazol-2-yl]- | 1.213; |
| | phenoxy}-acetamid | 372.4 |
| "A8" | 2-[2-(1-Benzyl-1*H*-benzimidazol-2-yl)-phenoxy]- | 1.654; |
| | acetamid | 358.2 |
| "A9" | 2-{2-[1-(2,6-Difluor-benzyl)-1*H*-benzimidazol-2- | 1.624 |
| | yl]-phenoxy}acetamid | 394.2 |
| "A10" | 2-{2-[1-(2,6-Dimethyl-benzyl)-1*H*-benzimidazol- | 1.396 |
| | 2-yl]-6-fluor-phenoxy}acetamid | 404.8 |
| "A11" | 2-{2-[1-(2,6-Dimethyl-benzyl)-1*H*-benzimidazol- | 1.347 |
| | 2-yl]-6-methoxy-phenoxy}-acetamid | 416.7 |
| "A12" | 2-{2-[1-(2,6-Dimethyl-benzyl)-1*H*-benzimidazol- | 1.336 |
| | 2-yl]-6-methyl-phenoxy}-acetamid | 400.8 |
| "A13" | 2-{2-[1-(2,6-Dimethyl-benzyl)-1*H*-benzimidazol- | 1.307 |
| | 2-yl]-5-methoxy-phenoxy}-acetamid | 416.7 |
| "A14" | 2-{2-[1-(2,6-Dimethyl-benzyl)-1*H*-benzimidazol- | 1.307 |
| | 2-yl]-5-methyl-phenoxy}-acetamid | 416.7 |
| "A15" | 2-{2-[1-(2,6-Dimethyl-benzyl)-1*H*-benzimidazol- | 1.324 |
| | 2-yl]-3-methoxy-phenoxy}-acetamid | 416.7 |
| "A16" | 2-{2-[1-(2,6-Dimethyl-benzyl)-1*H*-benzimidazol- | 1.252 |
| | 2-yl]-pyridin-3-yloxy}-acetamid | 387.7 |
| | 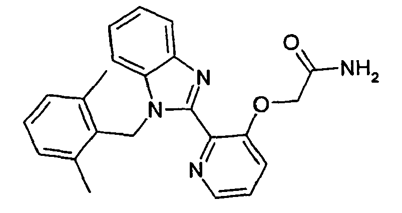 | |
| "A17" | 2-{2-[1-(2,6-Dimethyl-benzyl)-1*H*-benzimidazol- | 1.266 |
| | 2-yl]-5-fluor-phenoxy}-acetamide | 404.8 |
| "A18" | 2-{2-[5-Chlor-1-(2,6-dichlor-benzyl)-1*H*- | 1.668 |
| | benzimidazol-2-yl]-phenoxy}-acetamid | 461.6 |
| "A19" | 2-{2-[1-(2,6-Dichlor-benzyl)-5-fluor-1*H*- | 1.465 |
| | benzimidazol-2-yl]-phenoxy}-acetamid | 445.5 |
| "A20" | 2-{2-[1-(2-Chlor-6-fluor-benzyl)-1*H*- | 1.144 |
| | benzimidazol-2-yl]-phenoxy}-acetamid | 410.5 |
| "A21" | 2-{2-[7-Chlor-1-(2,6-dichlor-benzyl)-1*H*- | 1.811 |
| | benzimidazol-2-yl]-phenoxy}-acetamid | 461.6 |
| "A22" | 2-{2-[1-(2-Chlor-6-methyl-benzyl)-1*H*- | 2.001 |
| | benzimidazol-2-yl]-phenoxy}-acetamid | 406.5 |
| "A23" | 2-{2-[7-Brom-1-(2,6-dimethyl-benzyl)-1*H*- | 1.312 |
| | benzimidazol-2-yl]-phenoxy}-acetamid | 465.6 |
| | 2-{2-[7-Chlor-1-(2,6-dimethyl-benzyl)-1H- | 1.264 |
| "A24" | benzimidazol-2-yl]-phenoxy}-acetamid | 420.6 |
| | 2-{2-[1-(2,6-Dimethyl-benzoyl)-1*H*-benzimidazol- | 1.661 |
| | 2-yl]-phenoxy}-acetamid | 400.8 |
| "A25" | 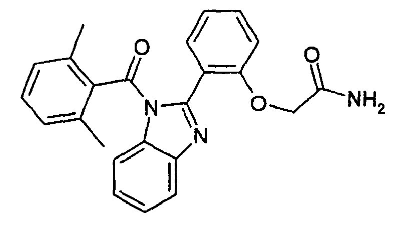 | |
| | 3-{2-[1-(2,6-Dimethyl-benzyl)-1H-benzimidazol- | 1.714 |
| | 2-yl]-phenoxy}-pyrrolidin-2-on | 412.7 |
| "A26" | 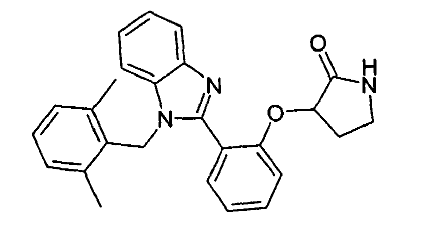 | |
| "A27" | 2-{2-[1-(2,6-Dimethyl-benzyl)-1*H*-benzimidazol- | 1.646 |
| | 2-yl]-phenoxy}-3-hydroxy-propionamid | 416.7 |
| | 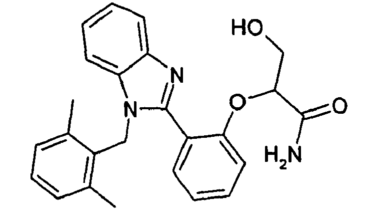 | |
| "A28" | 2-{2-[1-(2,6-Dimethyl-benzyl)-5,6-difluor-1*H*- | 1.77 |
| | benzimidazol-2-yl]-5-methoxy-3-methyl-phenoxy}-acetamid | 466.1 |
| "A29" | 2-{2-[1-(2,6-Dimethyl-benzyl)-6,7-difluor-1*H*- | 1.844 |
| | benzimidazol-2-yl]-phenoxy}-acetamid | 422.1 |
| "A30" | 2-{2-[1-(2,6-Dimethyl-benzyl)-6,7-difluor-1*H*- | 1.907 |
| | benzimidazol-2-yl]-5-methoxy-3-methyl-phenoxy}-acetamid | 466.1 |

### Beispiel 3

Die Herstellung von (5-{2-[1-(2,6-Dimethyl-benzyl)-1*H*-benzimidazol-2-yl]-phenyl}-[1,3,4]oxadiazol-2-yl)-methyl-amin ("A31 ") erfolgt wie nachstehend angegeben 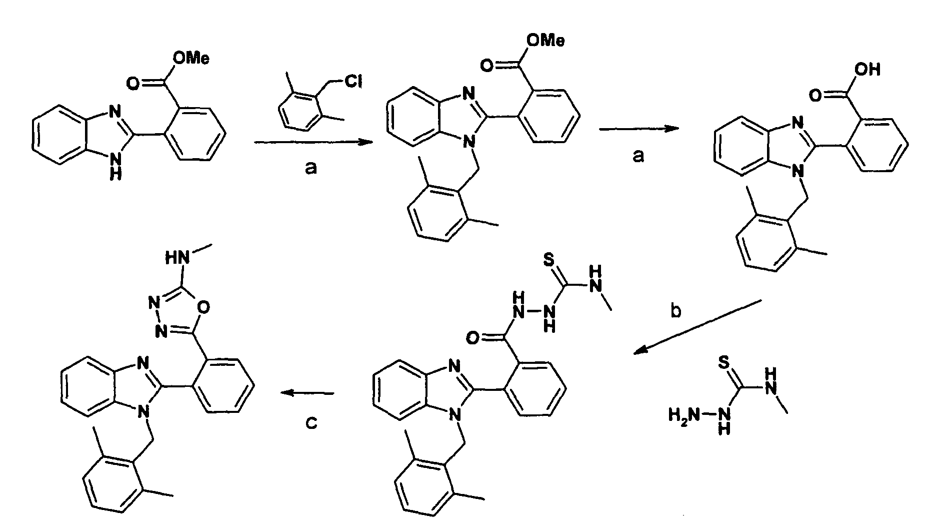
a) Eine Mischung aus 430 mg (1.7 mmol) 2-(1*H*-Benzimidazol-2-yl)-benzoesäure-methylester (Paulder et. al., J.Org.Chem.,(1969), 34(7) 2130-40), 266.3 mg (1.7 mmol) 2,6-Dimethylbenzylchlorid, 235 mg (1.7 mmol) Kaliumcarbonat und 28 mg Kaliumiodid in 5 ml Dimethylformamid wird 48 Stunden bei Raumtemperatur gerührt. Anschließend wird die Reaktionsmischung mit 20 ml Wasser versetzt, der ausgefallene Niederschlag abgetrennt, zweimal mit 10 ml Wasser gewaschen und an der Luft getrocknet. Nun wird der so erhaltene 2-[1-(2,6-Dimethyl-benzyl)-1*H-*benzimidazol-2-yl]-benzoesäure-methylester (MS: M+H = 371) in einer Mischung aus 4 ml Tetrahydrofuran, 6 ml Methanol und 2 ml Wasser gelöst und die Lösung nach Zugabe von 100 mg Lithiumhydroxyd 12 Stunden bei Raumtemperatur gerührt. Anschließend wird die Reaktionslösung im Vakuum eingeengt, der Rückstand in 5 ml Wasser gelöst, die Lösung mit 1 N Salzsäure angesäuert und dreimal mit je 10 ml Essigsäureethylester extrahiert. Nach dem Trocknen der vereinigten organischen Extrakte über Natriunsulfat und Abziehen des Lösungsmittels im Vakuum erhält man 460 mg 480 mg (75%) 2-[1-(2,6-Dimethyl-benzyl)-1*H-*benzimidazol-2-yl]-benzoesäure-methylester als gelben Harz. MS: M+H = 357.
b) Eine Mischung aus 0.46 g (1.3 mmol) 2-[1-(2,6-Dimethyl-benzyl)-1*H-*benzimidazol-2-yl]-benzoesäure, 154 mg (1.4 mmol) 4-Methyl-thiosemicarbazid, 300 mg (1.55 mmol) N-(3-Dimethylaminopropyl)-N'-ethylcarbo-diimid Hydrochlorid, 392 mg (1.68 mmol) 1-Hydroxybenzotriazol und 390 mg (3.87 mmol) 4-Methylmorpholin in 10 ml Dimethylformamid wird 12 Stunden bei Raumtemperatur gerührt und anschließend mit 50 ml Wasser versetzt. Der ausgefallene Niederschlag wird abgetrennt, zweimal mit 10 ml Wasser gewaschen und bis zu Massenkonstanz bei 50 °C im Vakuum getrocknet. Man erhält so 436 mg (88%) 1-[2-[1-(2,6-Dimethyl-benzyl)-1*H-*benzimidazol-2-yl]-benzoyl]-4-methyl-thiosemicarbazid als weißes Pulver. MS: M+H = 444.
c) Eine Lösung von 436 mg (0.98 mmol) der Verbindung aus Stufe b) und 344.6 mg (1.08 mmol) Quecksilber(II)-acetat wird 12 Stunden bei Raumtemperatur gerührt und anschließend über Kieselgur filtriert. Das Filtrat wird zu Trockne eingeengt und der Rückstand säulenchromatografisch (Kieselgel: Essigsäureethylester/Methanol 9/1) gereinigt. Man erhält so 232 mg (57.6%) "A31" als weißes Pulver; MS: M+H: 410; ¹H-NMR (d₆-DMSO): 8.1 ppm (m, 1H); 7.93 ppm (m, 1H); 7.84 ppm (d, 1H); 7.80 ppm (d, 1H); 7.22-6.65 ppm (m, 4H); 6.99 ppm (t, 1H); 6.73 ppm (d, 2H).

### Pharmakologische Daten

### Affinität zu Rezeptoren

**Tabelle 1**

| Verbindung Nr. | SGL T₁-IC₅₀ | SGLT₂-IC₅₀ |
|---|---|---|
| "A1" | C | A |
| "A2" | C | A |
| "A3" | | A |
| "A4" | C | A |
| "A5" | C | A |
| "A6" | C | C |
| "A7" | C | B |
| "A8" | C | C |
| "A9" | C | B |
| "A10" | C | A |
| "A11" | | B |
| "A12" | C | B |
| "A13" | C | B |
| "A14" | C | A |
| "A15" | C | A |
| "A16" | | C |
| "A17" | C | A |
| "A18" | C | C |
| "A19" | C | B |
| "A20" | C | B |
| "A21" | C | B |
| "A22" | C | B |
| "A23" | C | C |
| "A24" | B | A |
| "A25" | C | C |
| "A26" | C | B |
| "A27" | C | C |
| "A28" | C | B |
| "A29" | C | B |
| "A30" | B | A |
| "A31" | | A |

| | | |
|---|---|---|
| IC₅₀: 10 nM - 1 µM = A 1 µM - 10 µM = B > 10 µM = C | | |

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 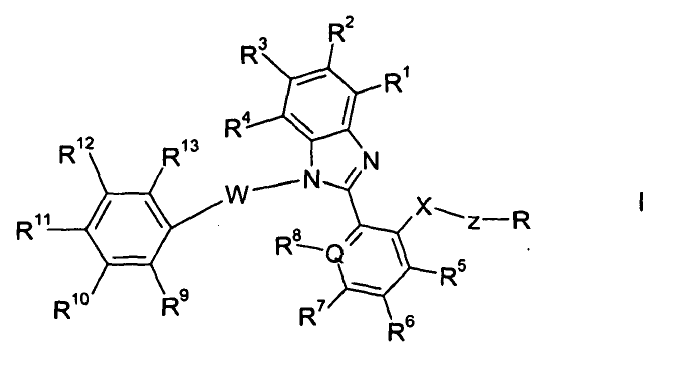 worin
R¹, R², R³, R⁴ jeweils unabhängig voneinander H, A oder Hal,
R⁵, R⁶, R⁷, R⁸ jeweils unabhängig voneinander H, A, Hal, OH oder OA, wobei R⁸ fehlt, falls Q = N,
R⁹, R¹⁰, R¹¹, R¹², R¹³ jeweils unabhängig voneinander H, A oder Hal, wobei einer der Reste R⁹, R¹⁰, R¹¹, R¹² oder R¹³ ≠ H ist,
R CO-NR¹⁸R¹⁹ oder Het,
Q N oder C,
X O oder NR¹⁴R¹⁵,
W (CR¹⁴R¹⁵)ₘ, CO, NR¹⁴R¹⁵, S(O)ₙ oder fehlt,
Z (CR²⁰R²¹)ₘ,
R¹⁴, R¹⁵ jeweils unabhängig voneinander H oder A,
R¹⁶, R¹⁷ jeweils unabhängig voneinander H oder A,
R¹⁸, R¹⁹ jeweils unabhängig voneinander H oder A,
R²⁰ H oder A,
R²¹ H, A oder CH₂OH,
R¹⁸ und R²¹ zusammen auch (CR¹⁶R¹⁷)ₚ,
Het einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch Hal, A, OH, OA, NR¹⁴R¹⁵ und/oder =O (Carbonylsauerstoff) substituiert sein kann,
A unverzweigtes oder verzweigtes Alkyl mit 1-10 C- Atomen, worin 1-7 H-Atome durch F ersetzt sein können,
Hal F, Cl, Br oder I,
m 0, 1, 2, 3 oder 4,
n 0, 1 oder 2,
p 1, 2 oder 3,
bedeuten,
sowie ihre pharmazeutisch verwendbaren Solvate, Salze und Stereoisomere, einschließlich deren Mischungen in allen Verhältnissen.

2. Verbindungen nach Anspruch 1, worin
A unverzweigtes oder verzweigtes Alkyl mit 1, 2, 3, 4, 5 oder 6 C-Atomen, worin 1-5 H-Atome durch F ersetzt sein können,
bedeutet,
sowie ihre pharmazeutisch verwendbaren Solvate, Salze und Stereoisomere, einschließlich deren Mischungen in allen Verhältnissen.

3. Verbindungen nach Anspruch 1 oder 2, worin
R¹, R², R³, R⁴ jeweils unabhängig voneinander H, CH₃, F oder Cl,
bedeuten,
sowie ihre pharmazeutisch verwendbaren Solvate, Salze und Stereoisomere, einschließlich deren Mischungen in allen Verhältnissen.

4. Verbindungen gemäß einem oder mehreren der Ansprüche 1-3, worin
R⁵, R⁶, R⁷, R⁸ jeweils unabhängig voneinander H, CH₃, Hal oder OCH₃,
wobei R⁸ fehlt, falls Q = N,
bedeuten,
sowie ihre pharmazeutisch verwendbaren Solvate, Salze und Stereoisomere, einschließlich deren Mischungen in allen Verhältnissen.

5. Verbindungen gemäß einem oder mehreren der Ansprüche 1-4, worin
R⁹, R¹⁰, R¹¹, R¹², R¹³ jeweils unabhängig voneinander H, CH₃, F oder Cl, wobei einer der Reste R⁹, R¹⁰, R¹¹, R¹² oder R¹³ ≠ H ist,
bedeuten,
sowie ihre pharmazeutisch verwendbaren Solvate, Salze und Stereoisomere, einschließlich deren Mischungen in allen Verhältnissen.

6. Verbindungen gemäß einem oder mehreren der Ansprüche 1-5, worin
R⁹, R¹³ jeweils unabhängig voneinander CH₃, F oder Cl,
R¹¹ H oder CH₃,
R¹⁰, R¹² H
bedeuten,
sowie ihre pharmazeutisch verwendbaren Solvate, Salze und Stereoisomere, einschließlich deren Mischungen in allen Verhältnissen.

7. Verbindungen gemäß einem oder mehreren der Ansprüche 1-6, worin
X O bedeutet,
sowie ihre pharmazeutisch verwendbaren Solvate, Salze und Stereoisomere, einschließlich deren Mischungen in allen Verhältnissen.

8. Verbindungen gemäß einem oder mehreren der Ansprüche 1-6, worin
W (CR¹⁴R¹⁵)ₘ oder CO bedeutet,
sowie ihre pharmazeutisch verwendbaren Solvate, Salze und Stereoisomere, einschließlich deren Mischungen in allen Verhältnissen.

9. Verbindungen gemäß einem oder mehreren der Ansprüche 1-8, worin
R¹⁴ , R¹⁵ H bedeuten,
sowie ihre pharmazeutisch verwendbaren Solvate. Salze und Stereoisomere, einschließlich deren Mischungen in allen Verhältnissen.

10. Verbindungen gemäß einem oder mehreren der Ansprüche 1-9, worin
R¹⁶, R¹⁷ H bedeuten,
sowie ihre pharmazeutisch verwendbaren Solvate, Salze und Stereoisomere, einschließlich deren Mischungen in allen Verhältnissen.

11. Verbindungen gemäß einem oder mehreren der Ansprüche 1-10, worin
R¹⁸, R¹⁹ H bedeuten,
sowie ihre pharmazeutisch verwendbaren Solvate, Salze und Stereoisomere, einschließlich deren Mischungen in allen Verhältnissen.

12. Verbindungen gemäß einem oder mehreren der Ansprüche 1-11, worin
R²⁰ H,
R²¹ H oder CH₂OH,
bedeuten,
sowie ihre pharmazeutisch verwendbaren Solvate, Salze und Stereoisomere, einschließlich deren Mischungen in allen Verhältnissen.

13. Verbindungen gemäß einem oder mehreren der Ansprüche 1-12, worin
Het einen ein- oder zweikernigen gesättigten, ungesättigten oder aromatischen Heterocyclus mit 1 bis 4 N-, O- und/oder S- Atomen, der ein- oder zweifach durch NR¹⁴R¹⁵ und/oder =O (Carbonylsauerstoff) substituiert sein kann,
bedeutet,
sowie ihre pharmazeutisch verwendbaren Solvate, Salze und Stereoisomere, einschließlich deren Mischungen in allen Verhältnissen.

14. Verbindungen gemäß einem oder mehreren der Ansprüche 1-13, worin
Het unsubstituiertes oder ein- oder zweifach durch NR¹⁴R¹⁵ und/oder =O (Carbonylsauerstoff) substituiertes Piperidinyl, Piperazinyl, Pyrrolidinyl, Morpholinyl, Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Indolyl, Benzo[1,3]dioxolyl, Indazolyl oder Benzo[2,1,3]thiadiazolyl
bedeutet,
sowie ihre pharmazeutisch verwendbaren Solvate, Salze und Stereoisomere, einschließlich deren Mischungen in allen Verhältnissen.

15. Verbindungen gemäß einem oder mehreren der Ansprüche 1-14, worin
A unverzweigtes oder verzweigtes Alkyl mit 1, 2, 3, 4, 5 oder 6 C-Atomen, worin 1-5 H-Atome durch F ersetzt sein können,
Het unsubstituiertes oder ein- oder zweifach durch NR¹⁴R¹⁵ und/oder =O (Carbonylsauerstoff) substituiertes Piperidinyl, Piperazinyl, Pyrrolidinyl, Morpholinyl, Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Indolyl, Benzo[1,3]dioxolyl, Indazolyl oder Benzo[2,1,3]thiadiazolyl,
R¹, R², R³, R⁴ jeweils unabhängig voneinander H, CH₃, F oder Cl,
R⁵, R⁶, R⁷, R⁸ jeweils unabhängig voneinander H, CH₃, Hal oder OCH₃,
wobei R⁸ fehlt, falls Q = N,
R⁹, R¹³ jeweils unabhängig voneinander CH₃, F oder Cl,
R CO-NR¹⁸R¹⁹ oder Het,
R¹¹ H oder CH₃,
R¹⁰, R¹² H,
Q N oder C,
X O,
W (CR¹⁴R¹⁵)ₘ oder CO,
Z (CR²⁰R²¹)*ₘ*,
R¹⁴, R¹⁵ H,
R¹⁶, R¹⁷ H,
R¹⁸, R¹⁹ H,
R²⁰ H,
R²¹ H oder CH₂OH,
R¹⁸ und R²¹ zusammen auch (CR¹⁶R¹⁷)ₚ,
m 0, 1 oder 2,
p 1 oder 2,
bedeuten,
sowie ihre pharmazeutisch verwendbaren Solvate, Salze und Stereoisomere, einschließlich deren Mischungen in allen Verhältnissen.

16. Verbindungen nach Anspruch 1 ausgewählt aus der Gruppe
| Nr. | Name |
|---|---|
| "A1" | 2-{2-[1-(2,6-Dimethyl-benzyl)-6-fluor-1*H*-benzimidazol-2-yl]-5-methoxy-3-methyl-phenoxy}-acetamid |
| "A2" | 2-{2-[1-(2,6-Dimethyl-benzyl)-1*H*-benzimidazol-2-yl]-phenoxy}-acetamid |
| "A3" | 2-{2-[1-(2,6-Dimethyl-benzyl)-1*H*-benzimidazol-2-yl]-3-fluor-phenoxy}-acetamid |
| | 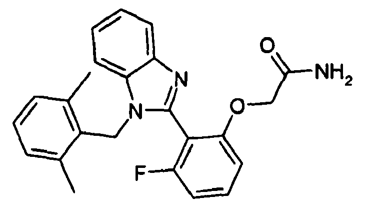 |
| "A4" | 2-{2-[1-(2,6-Dimethyl-benzyl)-6-fluor-1*H*-benzimidazol-2-yl]-phenoxy}-acetamid |
| | 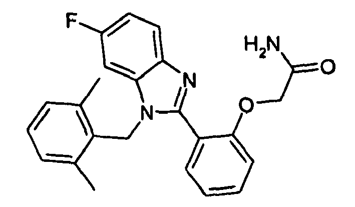 |
| "A5" | 2-{2-[1-(2,6-Dimethyl-benzyl)-5,6-difluor-1*H*-benzimidazol-2-yl]-phenoxy}-acetamid |
| "A6" | 2-{2-[6-Fluor-1-(2,4,6-trimethyl-benzyl)-1*H*-benzimidazol-2-yl]-phenoxy}-acetamid |
| "A7" | 2-{2-[1-(2-Methyl-benzyl)-1*H*-benzimidazol-2-yl]-phenoxy}-acetamid |
| "A8" | 2-[2-(1-Benzyl-1*H*-benzimidazol-2-yl)-phenoxy]-acetamid |
| "A9" | 2-{2-[1-(2,6-Difluor-benzyl)-1*H*-benzimidazol-2-yl]-phenoxy}-acetamid |
| "A10" | 2-{2-[1-(2,6-Dimethyl-benzyl)-1*H*-benzimidazol-2-yl]-6-fluor-phenoxy}-acetamid |
| "A11" | 2-{2-[1-(2,6-Dimethyl-benzyl)-1*H*-benzimidazol-2-yl]-6-methoxy-phenoxy}-acetamid |
| "A12" | 2-{2-[1-(2,6-Dimethy)-benzyl)-1*H*-benzimidazol-2-yl]-6-methyl-phenoxy}-acetamid |
| "A13" | 2-{2-[1-(2,6-Dimethyl-benzyl)-1*H*-benzimidazol-2-yl]-5-methoxy-phenoxy}-acetamid |
| "A14" | 2-{2-[1-(2,6-Dimethyl-benzyl)-1*H*-benzimidazol-2-yl]-5-methyl-phenoxy}-acetamid |
| "A15" | 2-{2-[1-(2,6-Dimethyl-benzyl)-1*H*-benzimidazol-2-yl]-3-methoxy-phenoxy}-acetamid |
| "A16" | 2-{2-[1-(2,6-Dimethyl-benzyl)-1*H*-benzimidazol-2-yl]-pyridin-3-yloxy}-acetamid |
| | 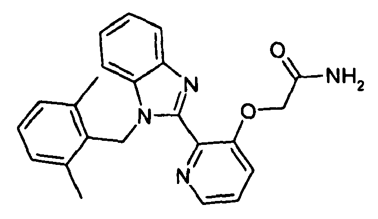 |
| "A17" | 2-{2-[1-(2,6-Dimethyl-benzyl)-1*H*-benzimidazol-2-yl]-5-fluor-phenoxy}-acetamide |
| "A18" | 2-{2-[5-Chlor-1-(2,6-dichlor-benzyl)-1*H*-benzimidazol-2-yl]-phenoxy}-acetamid |
| "A19" | 2-{2-[1-(2,6-Dichlor-benzyl)-5-fluor-1*H*-benzimidazol-2-yl]-phenoxy}-acetamid |
| "A20" | 2-{2-[1-(2-Chlor-6-fluor-benzyl)-1*H*-benzimidazol-2-yl]-phenoxy}-acetamid |
| "A21" | 2-{2-[7-Chlor-1-(2,6-dichlor-benzyl)-1*H*-benzimidazol-2-yl]-phenoxy}-acetamid |
| "A22" | 2-{2-[1-(2-Chlor-6-methyl-benzyl)-1*H*-benzimidazol-2-yl]-phenoxy}-acetamid |
| "A23" | 2-{2-[7-Brom-1-(2,6-dimethyl-benzyl)-1*H*-benzimidazol-2-yl]-phenoxy}-acetamid |
| "A24" | 2-{2-[7-Chlor-1-(2,6-dimethyl-benzyl)-1*H*-benzimidazol-2-yl]-phenoxy}-acetamid |
| "A25" | 2-{2-[1-(2,6-Dimethyl-benzoyl)-1*H*-benzimidazol-2-yl]-phenoxy}-acetamid |
| | 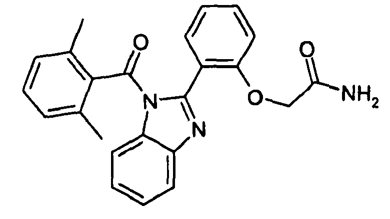 |
| "A26" | 3-{2-[1-(2,6-Dimethyl-benzyl)-1H-benzimidazol-2-yl]-phenoxy}-pyrrolidin-2-on |
| | 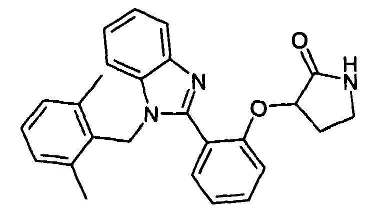 |
| "A27" | 2-{2-[1-(2,6-Dimethyl-benzyl)-1*H*-benzimidazol-2-yl]-phenoxy}-3-hydroxy-propionamid |
| | 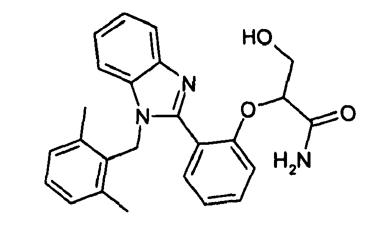 |
| "A28" | 2-{2-[1-(2,6-Dimethyl-benzyl)-5,6-difluor-1*H*-benzimidazol-2-yl]-5-methoxy-3-methyl-phenoxy}-acetamid |
| "A29" | 2-{2-[1-(2,6-Dimethyl-benzyl)-6,7-difluor-1*H*-benzimidazol-2-yl]-phenoxy}-acetamid |
| "A30" | 2-{2-[1-(2,6-Dimethyl-benzyl)-6,7-difluor-1*H*-benzimidazol-2-yl]-5-methoxy-3-methyl-phenoxy}-acetamid |
sowie ihre pharmazeutisch verwendbaren Solvate, Salze und Stereoisomere, einschließlich deren Mischungen in allen Verhältnissen.

17. Verfahren zur Herstellung von Verbindungen der Formel I nach den Ansprüchen 1-16 sowie ihrer pharmazeutisch verwendbaren Solvate, Salze und Stereoisomere, **dadurch gekennzeichnet, daß** man
a) eine Verbindung der Formel II 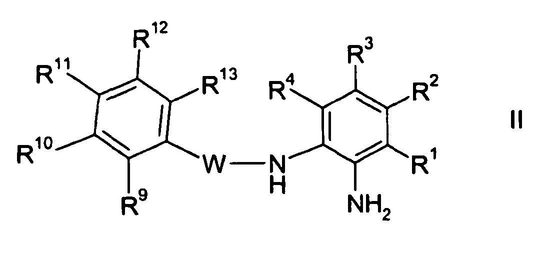 worin
R¹, R², R³, R⁴, R⁹, R¹⁰, R¹¹, R¹², R¹³ und W die in Anspruch 1 angegebenen Bedeutungen haben,
mit einer Verbindung der Formel III 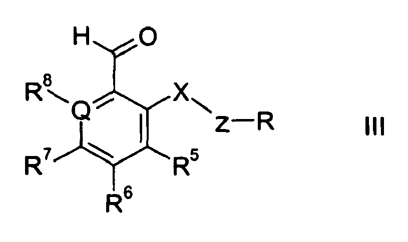 worin
R⁵, R⁶, R⁷, R⁸, R, Q, W und Z die in Anspruch 1 angegebenen Bedeutungen haben,
umsetzt,
oder
b) eine Verbindung der Formel IV 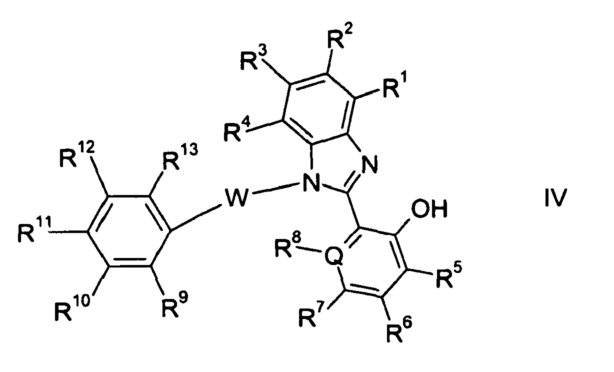 worin
R¹, R², R³, R⁴, R⁵, R⁶, R⁷, R⁸, R⁹, R¹⁰, R¹¹, R¹², R¹³ Q und W die in Patentanspruch 1 angegebenen Bedeutungen haben,
mit einer Verbindung der Formel V
L-Z-R V
worin Z und R die in Patentanspruch 1 angegebenen Bedeutungen haben und
L Cl, Br, I oder eine freie oder reaktionsfähig funktionell abgewandelte OH-Gruppe bedeutet,
umsetzt,
und/oder eine Base oder Säure der Formel I in eines ihrer Salze umwandelt.

18. Arzneimittel, enthaltend mindestens eine Verbindung der Formel I nach einem oder mehreren der Ansprüche 1 bis 16 und/oder ihre pharmazeutisch verwendbaren Solvate, Salze und Stereoisomere, einschließlich deren Mischungen in allen Verhältnissen, sowie gegebenenfalls Träger- und/oder Hilfsstoffe.

19. Arzneimittel enthaltend mindestens eine Verbindung der Formel I gemäß einem oder mehreren der Ansprüche 1 bis 16 und/oder ihre pharmazeutisch verwendbaren Solvate und Stereoisomere, einschließlich deren Mischungen in allen Verhältnissen, und mindestens einen weiteren Arzneimittelwirkstoff.

20. Verwendung von Verbindungen gemäß einem oder mehreren der Ansprüche 1 bis 16 und/oder ihre physiologisch unbedenklichen Salze, Salze und Solvate zur Herstellung eines Arzneimittels zur Behandlung des Typ 1 und Typ 2 Diabetes.

21. Verwendung von Verbindungen gemäß einem oder mehreren der Ansprüche 1 bis 16 und/oder ihre physiologisch unbedenklichen Salze, Salze und Solvate zur Herstellung eines Arzneimittels zur Blutzuckersenkung.

22. Verwendung von Verbindungen gemäß einem oder mehreren der Ansprüche 1 bis 16 und/oder ihre physiologisch unbedenklichen Salze, Salze und Solvate und einem weiteren Arzneimittelwirkstoff zur Herstellung eines Arzneimittels zur Behandlung des Typ 1 und Typ 2 Diabetes.

23. Set (Kit), bestehend aus getrennten Packungen von
(a) einer wirksamen Menge an einer Verbindung der Formel I gemäß einem oder mehreren der Ansprüche 1 bis 16 und/oder ihrer pharmazeutisch verwendbaren Solvate, Salze und Stereoisomere, einschließlich deren Mischungen in allen Verhältnissen,
und
(b) einer wirksamen Menge eines weiteren Arzneimittelswirkstoffs.

24. Verwendung von Verbindungen gemäß einem oder mehreren der Ansprüche 1 bis 16 und/oder ihre physiologisch unbedenklichen Salze, Salze und Solvate und einem weiteren Arzneimittelwirkstoff zur Herstellung eines Arzneimittels zur Blutzuckersenkung.

25. (5-{2-[1-(2,6-Dimethyl-benzyl)-1H-benzimidazol-2-yl]-phenyl}-[1,3,4]-oxadiazol-2-yl)-methyl-amin,sowie seine pharmazeutisch verwendbaren Salze.

## Claims

1. Compounds of the formula I 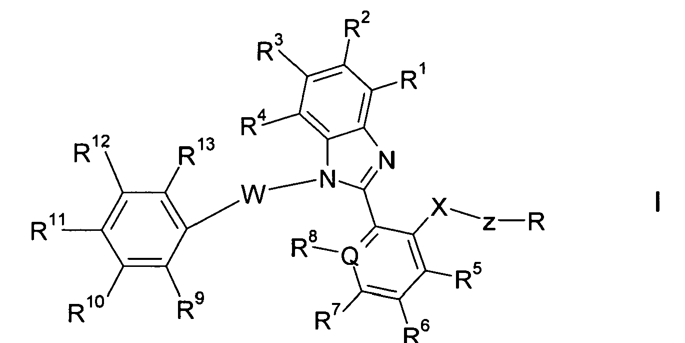 in which
R¹, R², R³, R⁴ each, independently of one another, denote H, A or Hal,
R⁵, R⁶, R⁷, R⁸ each, independently of one another, denote H, A, Hal, OH or OA, where R⁸ is absent if Q = N,
R⁹, R¹⁰, R¹¹, R¹², R¹³ each, independently of one another, denote H, A or Hal, where one of the radicals R⁹, R¹⁰, R¹¹, R¹² or R¹³ is ≠ H,
R denotes CO-NR¹⁸R¹⁹ or Het,
Q denotes N or C,
X denotes O or NR¹⁴R¹⁵,
W denotes (CR¹⁴R¹⁵)ₘ, CO, NR¹⁴R¹⁵, S(O)ₙ or is absent,
Z denotes (CR²⁰R²¹)ₘ,
R¹⁴, R¹⁵ each, independently of one another, denote H or A,
R¹⁶, R¹⁷ each, independently of one another, denote H or A,
R¹⁸, R¹⁹ each, independently of one another, denote H or A,
R²⁰ denotes H or A,
R²¹ denotes H, A or CH₂OH,
R¹⁸ and R²¹ together also denote (CR¹⁶R¹⁷)ₚ,
Het denotes a mono- or bicyclic saturated, unsaturated or aromatic heterocycle having 1 to 4 N, O and/or S atoms, which may be unsubstituted or mono-, di- or trisubstituted by Hal, A, OH, OA, NR¹⁴R¹⁵ and/or =O (carbonyl oxygen),
A denotes unbranched or branched alkyl having 1-10 C atoms, in which 1-7 H atoms may be replaced by F,
Hal denotes F, CI, Br or I,
m denotes 0, 1, 2, 3 or 4,
n denotes 0, 1 or 2,
p denotes 1, 2 or 3,
and pharmaceutically usable solvates, salts and stereoisomers thereof, including mixtures thereof in all ratios.

2. Compounds according to Claim 1 in which A denotes unbranched or branched alkyl having 1, 2, 3, 4, 5 or 6 C atoms, in which 1-5 H atoms may be replaced by F, and pharmaceutically usable solvates, salts and stereoisomers thereof, including mixtures thereof in all ratios.

3. Compounds according to Claim 1 or 2 in which
R¹, R², R³, R⁴ each, independently of one another, denote H, CH₃, F or CI,
and pharmaceutically usable solvates, salts and stereoisomers thereof, including mixtures thereof in all ratios.

4. Compounds according to one or more of Claims 1-3 in which
R⁵ R⁶, R⁷, R⁸ each, independently of one another, denote H, CH₃, Hal or OCH₃,
where R⁸ is absent if Q = N,
and pharmaceutically usable solvates, salts and stereoisomers thereof, including mixtures thereof in all ratios.

5. Compounds according to one or more of Claims 1-4 in which
R⁹ R¹⁰, R¹¹, R¹², R¹³ each, independently of one another, denote H, CH₃, F or CI,
where one of the radicals R⁹, R¹⁰, R¹¹, R¹² or R¹³ is ≠ H,
and pharmaceutically usable solvates, salts and stereoisomers thereof, including mixtures thereof in all ratios.

6. Compounds according to one or more of Claims 1-5 in which
R⁹, R¹³ each, independently of one another, denote CH₃, F or Cl,
R¹¹ denotes H or CH₃,
R¹⁰, R¹² denote H,
and pharmaceutically usable solvates, salts and stereoisomers thereof, including mixtures thereof in all ratios.

7. Compounds according to one or more of Claims 1-6 in which
X denotes O,
and pharmaceutically usable solvates, salts and stereoisomers thereof, including mixtures thereof in all ratios.

8. Compounds according to one or more of Claims 1-6 in which
W denotes (CR¹⁴R¹⁵)ₘ or CO,
and pharmaceutically usable solvates, salts and stereoisomers thereof, including mixtures thereof in all ratios.

9. Compounds according to one or more of Claims 1-8 in which
R¹⁴, R¹⁵ denote H,
and pharmaceutically usable solvates, salts and stereoisomers thereof, including mixtures thereof in all ratios.

10. Compounds according to one or more of Claims 1-9 in which
R¹⁶, R¹⁷ denote H,
and pharmaceutically usable solvates, salts and stereoisomers thereof, including mixtures thereof in all ratios.

11. Compounds according to one or more of Claims 1-10 in which
R¹⁸, R¹⁹ denote H,
and pharmaceutically usable solvates, salts and stereoisomers thereof, including mixtures thereof in all ratios.

12. Compounds according to one or more of Claims 1-11 in which
R²⁰ denotes H,
R²¹ denotes H or CH₂OH,
and pharmaceutically usable solvates, salts and stereoisomers thereof, including mixtures thereof in all ratios.

13. Compounds according to one or more of Claims 1-12 in which
Het denotes a mono- or bicyclic saturated, unsaturated or aro- matic heterocycle having 1 to 4 N, O and/or S atoms, which may be mono- or disubstituted by NR¹⁴R¹⁵ and/or =O (car- bonyl oxygen),
and pharmaceutically usable solvates, salts and stereoisomers thereof, including mixtures thereof in all ratios.

14. Compounds according to one or more of Claims 1-13 in which
Het denotes piperidinyl, piperazinyl, pyrrolidinyl, morpholinyl, furyl, thienyl, pyrrolyl, imidazolyl, pyrazolyl, oxazolyl, isoxazo- lyl, thiazolyl, isothiazolyl, pyridyl, pyrimidinyl, triazolyl, tetrazo- lyl, oxadiazolyl, thiadiazolyl, pyridazinyl, pyrazinyl, benzimida- zolyl, benzotriazolyl, indolyl, benzo-1,3-dioxolyl, indazolyl or benzo-2,1,3-thiadiazolyl, each of which is unsubstituted or mono- or disubstituted by NR¹⁴R¹⁵ and/or =O (carbonyl oxy- gen),
and pharmaceutically usable solvates, salts and stereoisomers thereof, including mixtures thereof in all ratios.

15. Compounds according to one or more of Claims 1-14 in which
A denotes unbranched or branched alkyl having 1, 2, 3, 4, 5 or 6 C atoms, in which 1-5 H atoms may be replaced by F,
Het denotes piperidinyl, piperazinyl, pyrrolidinyl, morpho- linyl, furyl, thienyl, pyrrolyl, imidazolyl, pyrazolyl, oxazolyl, isoxazolyl, thiazolyl, isothiazolyl, pyridyl, pyrimidinyl, triazolyl, tetrazolyl, oxadiazolyl, thiadiazo- lyl, pyridazinyl, pyrazinyl, benzimidazolyl, benzotri- azolyl, indolyl, benzo-1,3-dioxolyl, indazolyl or benzo- 2,1,3-thiadiazolyl, each of which is unsubstituted or mono- or disubstituted by NR¹⁴R¹⁵ and/or =O (car- bonyl oxygen),
R¹, R², R³, R⁴ each, independently of one another, denote H, CH₃, F or Cl,
R⁵, R⁶, R⁷, R⁸ each, independently of one another, denote H, CH₃, Hal or OCH₃, where R⁸ is absent if Q = N,
R⁹, R¹³ each, independently of one another, denote CH₃, F or Cl,
R denotes CO-NR¹⁸R¹⁹ or Het,
R¹¹ denotes H or CH₃,
R¹⁰ R¹² denote H,
Q denotes N or C,
X denotes O,
W denotes (CR¹⁴R15)ₘ or CO,
Z denotes (CR²⁰R²¹)ₘ,
R¹⁴ R¹⁵ denote H,
R¹⁶ R¹⁷ denote H,
R¹⁸ R¹⁹ denote H,
R²⁰ denotes H,
R²¹ denotes H or CH₂OH,
R¹⁸ and R²¹ together also denote (CR¹⁶R¹⁷)ₚ,
m denotes 0, 1 or 2,
p denotes 1 or 2,
and pharmaceutically usable solvates, salts and stereoisomers thereof, including mixtures thereof in all ratios.

16. Compounds according to Claim 1 selected from the group
| No. | Name |
|---|---|
| "A1" | 2-{2-[1-(2,6-Dimethylbenzyl)-6-fluoro-1*H*-benzimidazol-2-yl]-5-methoxy-3-methylphenoxy}acetamide |
| "A2" | 2-{2-[1-(2,6-Dimethylbenzyl)-1*H*-benzimidazol-2-yl]-phenoxy}acetamide |
| "A3" | 2-{2-[1-(2,6-Dimethylbenzyl)-1*H*-benzimidazol-2-yl]-3-fluorophenoxy}acetamide |
| | 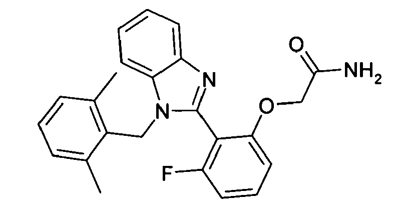 |
| "A4" | 2-{2-[1-(2,6-Dimethylbenzyl)-6-fluoro-1*H*-benzimidazol-2-yl]phenoxy}acetamide |
| | 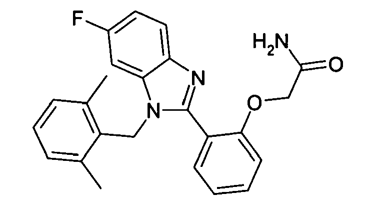 |
| "A5" | 2-{2-[1-(2,6-Dimethylbenzyl)-5,6-difluoro-1*H*-benzimida-zol-2-yl]phenoxy}acetamide |
| "A6" | 2-{2-[6-Fluoro-1-(2,4,6-trimethylbenzyl)-1*H*-benzimidazol-2-yl]phenoxy}acetamide |
| "A7" | 2-{2-[1-(2-Methylbenzyl)-1*H*-benzimidazol-2-yl]phenoxy}-acetamide |
| "A8" | 2-[2-(1-Benzyl-1*H*-benzimidazol-2-yl)phenoxy]acetamide |
| "A9" | 2-{2-[1-(2,6-Difluorobenzyl)-1*H*-benzimidazol-2-yl]-phenoxy}acetamide |
| "A10" | 2-{2-[1-(2,6-Dimethylbenzyl)-1*H*-benzimidazol-2-yl]-6-fluorophenoxy}acetamide |
| "A11" | 2-{2-[1-(2,6-Dimethylbenzyl)-1*H*-benzimidazol-2-yl]-6-methoxyphenoxy}acetamide |
| "A12" | 2-{2-[1-(2,6-Dimethylbenzyl)-1*H*-benzimidazol-2-yl]-6-methylphenoxy}acetamide |
| "A13" | 2-{2-[1-(2,6-Dimethylbenzyl)-1*H*-benzimidazol-2-yl]-5-methoxyphenoxy}acetamide |
| "A14" | 2-{2-[1-(2,6-Dimethylbenzyl)-1*H*-benzimidazol-2-yl]-5-methylphenoxy}acetamide |
| "A15" | 2-{2-[1-(2,6-Dimethylbenzyl)-1*H*-benzimidazol-2-yl]-3-methoxyphenoxy}acetamide |
| "A16" | 2-{2-[1-(2,6-Dimethylbenzyl)-1*H*-benzimidazol-2-yl]pyri-din-3-yloxy}acetamide |
| | 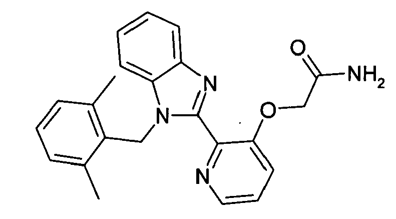 |
| "A17" | 2-{2-[1-(2,6-Dimethylbenzyl)-1*H*-benzimidazol-2-yl]-5-fluorophenoxy}acetamide |
| "A18" | 2-{2-[5-Chloro-1-(2,6-dichlorobenzyl)-1*H*-benzimidazol-2-yl]phenoxy}acetamide |
| "A19" | 2-{2-[1-(2,6-Dichlorobenzyl)-5-fluoro-1*H*-benzimidazol-2-yl]phenoxy}acetamide |
| "A20" | 2-{2-[1-(2-Chloro-6-fluorobenzyl)-1*H*-benzimidazol-2-yl]-phenoxy}acetamide |
| "A21" | 2-{2-[7-Chloro-1-(2,6-dichlorobenzyl)-1*H*-benzimidazol-2-yl]phenoxy}acetamide |
| "A22" | 2-{2-[1-(2-Chloro-6-methylbenzyl)-1*H*-benzimidazol-yl]phenoxy}acetamide |
| "A23" | 2-{2-[7-Bromo-1-(2,6-dimethylbenzyl)-1*H*-benzimidazol-2-yl]phenoxy}acetamide |
| "A24" | 2-{2-[7-Chloro-1-(2,6-dimethylbenzyl)-1*H*-benzimidazol-2-yl]phenoxy}acetamide |
| "A25" | 2-{2-[1-(2,6-Dimethylbenzoyl)-1*H*-benzimidazol-2-yl]-phenoxy}acetamide |
| | 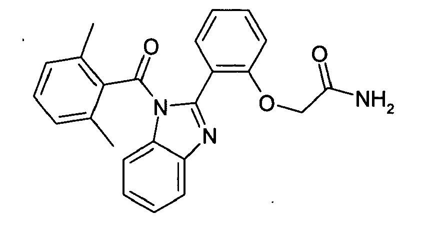 |
| "A26" | 3-{2-[1-(2,6-Dimethylbenzyl)-1*H*-benzimidazol-2-yl]-phenoxy}pyrrolidin-2-one |
| | 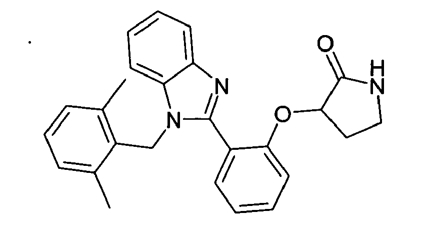 |
| "A27" | 2-{2-[1-(2,6-Dimethylbenzyl)-1*H*-benzimidazol-2-yl]-phenoxy}-3-hydroxypropionamide |
| | 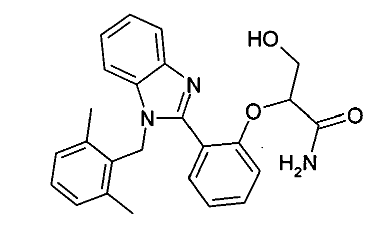 |
| "A28" | 2-{2-[1-(2,6-Dimethylbenzyl)-5,6-difluoro-1*H*-benzimida-zol-2-yl]-5-methoxy-3-methylphenoxy}acetamide |
| "A29" | 2-{2-[1-(2,6-Dimethylbenzyl)-6,7-difluoro-1*H*-benzimida-zol-2-yl]phenoxy}acetamide |
| "A30" | 2-{2-[1-(2,6-Dimethylbenzyl)-6,7-difluoro-1*H*-benzimida-zol-2-yl]-5-methoxy-3-methylphenoxy}acetamide |
and pharmaceutically usable solvates, salts and stereoisomers thereof, including mixtures thereof in all ratios.

17. Process for the preparation of compounds of the formula I according to Claims 1-16 and pharmaceutically usable solvates, salts and stereoisomers thereof, **characterised in that**
a) a compound of the formula II 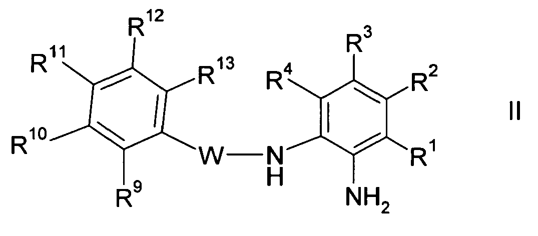 in which
R¹, R², R³, R⁴, R⁹, R¹⁰, R¹¹, R¹², R¹³ and W have the meanings indicated in Claim 1,
is reacted with a compound of the formula III 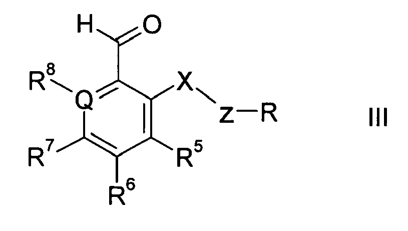 in which
R⁵, R⁶, R⁷, R⁸, R, Q, X and Z have the meanings indicated in Claim 1,
or
b) a compound of the formula IV 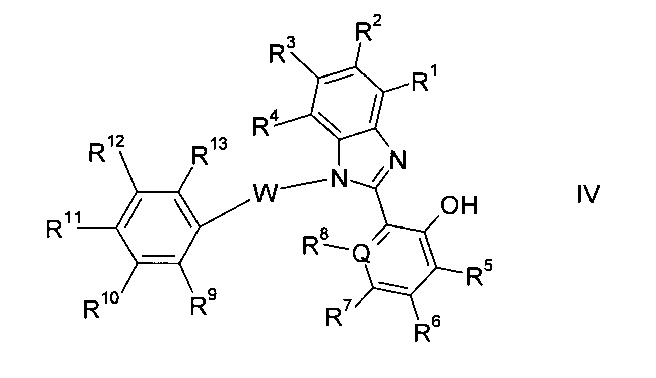 in which
R¹, R², R³, R⁴, R⁵, R⁶, R⁷, R⁸, R⁹, R¹⁰, R¹¹, R¹², R¹³, Q and W have the meanings indicated in Patent Claim 1,
is reacted with a compound of the formula V
L-Z-R V
in which Z and R have the meanings indicated in Patent Claim 1, and
L denotes Cl, Br, I or a free or reactively functionally modified OH group,
and/or
a base or acid of the formula I is converted into one of its salts.

18. Medicaments comprising at least one compound of the formula I according to one or more of Claims 1 to 16 and/or pharmaceutically usable solvates, salts and stereoisomers thereof, including mixtures thereof in all ratios, and optionally excipients and/or adjuvants.

19. Medicaments comprising at least one compound of the formula I according to one or more of Claims 1 to 16 and/or pharmaceutically usable solvates and stereoisomers thereof, including mixtures thereof in all ratios, and at least one further medicament active ingredient.

20. Use of compounds according to one or more of Claims 1 to 16 and/or physiologically acceptable salts, salts and solvates thereof for the preparation of a medicament for the treatment of type 1 and type 2 diabetes.

21. Use of compounds according to one or more of Claims 1 to 16 and/or physiologically acceptable salts, salts and solvates thereof for the preparation of a medicament for lowering blood sugar.

22. Use of compounds according to one or more of Claims 1 to 16 and/or physiologically acceptable salts, salts and solvates thereof and a further medicament active ingredient for the preparation of a medicament for the treatment of type 1 and type 2 diabetes.

23. Set (kit) consisting of separate packs of
(a) an effective amount of a compound of the formula I according to one or more of Claims 1 to 16 and/or pharmaceutically usable solvates, salts and stereoisomers thereof, including mixtures thereof in all ratios,
and
(b) an effective amount of a further medicament active ingredient.

24. Use of compounds according to one or more of Claims 1 to 16 and/or physiologically acceptable salts, salts and solvates thereof and a further medicament active ingredient for the preparation of a medicament for lowering blood sugar.

25. (5-{2-[1-(2,6-Dimethylbenzyl)-1*H*-benzimidazol-2-yl]phenyl}-1,3,4-oxadiazol-2-yl)methylamine, and pharmaceutically usable salts thereof.

## Revendications

1. Composés de formule I 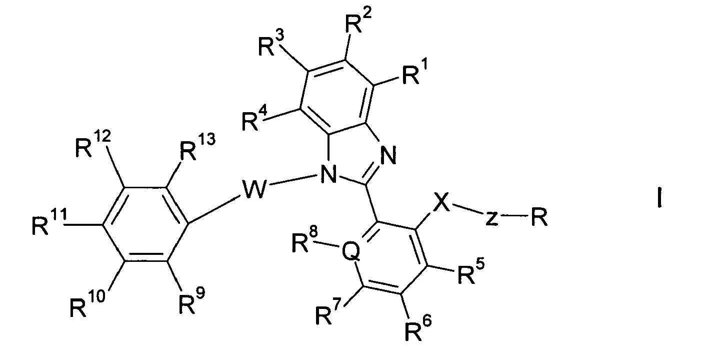 dans laquelle
R¹, R², R³, R⁴ désignent chacun, indépendamment les uns des autres, H, A ou Hal,
R⁵, R⁶, R⁷, R⁸ désignent chacun, indépendamment les uns des autres, H, A, Hal, OH ou OA, où R⁸ est absent si Q = N,
R⁹ R¹⁰, R¹¹ R¹², R¹³ désignent chacun, indépendamment les uns des autres, H, A ou Hal, où l'un des radicaux R⁹, R¹⁰ R¹¹, R¹² ou R¹³ est H,
R désigne CO-NR¹⁸R¹⁹ ou Hét,
Q désigne N ou C,
X désigne O ou NR¹⁴R¹⁵,
W désigne (CR¹⁴R¹⁵)ₘ, CO, NR¹⁴R¹⁵, S(O)ₙ ou est absent,
Z désigne (CR²⁰R²¹)ₘ,
R¹⁴, R¹⁵ désignent chacun, indépendamment les uns des autres, H ou A,
R¹⁶, R¹⁷ désignent chacun, indépendamment les uns des autres, H ou A,
R¹⁸, R¹⁹ désignent chacun, indépendamment les uns des autres, H ou A,
R²⁰ désigne H ou A,
R²¹ désigne H, A ou CH₂OH,
R¹⁸ et R²¹ désignent aussi ensemble (CR¹⁶R¹⁷)ₚ,
Hét désigne un hétérocycle mono- ou bicyclique saturé, insaturé ou aromatique ayant de 1 à 4 atomes de N, O et/ou S, pouvant être non substi- tué ou mono-, di- ou trisubstitué par Hal, A, OH, OA, NR¹⁴R¹⁵ et/ou =O (oxygène carbonyle),
A désigne alkyle linéaire ou ramifié ayant 1-10 atomes de C, où 1-7 atomes de H peuvent être remplacés par F,
Hal désigne F, Cl, Br ou I,
m désigne 0, 1, 2, 3 ou 4,
n désigne 0, 1 ou 2,
p désigne 1, 2 ou 3,
et les solvats, sels et stéréoisomères pharmaceutiquement utilisables de ceux-ci, y compris des mélanges de ceux-ci en toutes proportions.

2. Composés selon la revendication 1, dans lesquels
A désigne alkyle linéaire ou ramifié ayant 1, 2, 3, 4, 5 ou 6 atomes de C, où 1-5 atomes de H peuvent être remplacés par F,
et les solvats, sels et stéréoisomères pharmaceutiquement utilisables de ceux-ci, y compris des mélanges de ceux-ci en toutes proportions.

3. Composés selon la revendication 1 ou 2, dans lesquels
R¹, R², R³, R⁴ désignent chacun, indépendamment les uns des autres, H, CH₃, F ou Cl,
et les solvats, sels et stéréoisomères pharmaceutiquement utilisables de ceux-ci, y compris des mélanges de ceux-ci en toutes proportions.

4. Composés selon l'une ou plusieurs des revendications 1-3, dans lesquels
R⁵, R⁶, R⁷, R⁸ désignent chacun, indépendamment les uns des autres, H, CH₃, Hal ou OCH₃,
où R⁸ est absent si Q = N, et les solvats, sels et stéréoisomères pharmaceutiquement utilisables de ceux-ci, y compris des mélanges de ceux-ci en toutes proportions.

5. Composés selon l'une ou plusieurs des revendications 1-4, dans lesquels
R⁹, R¹⁰, R¹¹, R¹², R¹³ désignent chacun, indépendamment les uns des autres, H, CH₃, F ou Cl,
où l'un des radicaux R⁹, R¹⁰, R¹¹, R¹² ou R¹³ est ≠ H,
et les solvats, sels et stéréoisomères pharmaceutiquement utilisables de ceux-ci, y compris des mélanges de ceux-ci en toutes proportions.

6. Composés selon l'une ou plusieurs des revendications 1-5, dans lesquels
R⁹, R¹³ désignent chacun, indépendamment les uns des autres, CH₃, F ou Cl,
R¹¹ désigne H ou CH₃,
R¹⁰, R¹² désignent H,
et les solvats, sels et stéréoisomères pharmaceutiquement utilisables de ceux-ci, y compris des mélanges de ceux-ci en toutes proportions.

7. Composés selon l'une ou plusieurs des revendications 1-6, dans lesquels
X désigne O,
et les solvats, sels et stéréoisomères pharmaceutiquement utilisables de ceux-ci, y compris des mélanges de ceux-ci en toutes proportions.

8. Composés selon l'une ou plusieurs des revendications 1-6, dans lesquels
W désigne (CR¹⁴R¹⁵)ₘ ou CO,
et les solvats, sels et stéréoisomères pharmaceutiquement utilisables de ceux-ci, y compris des mélanges de ceux-ci en toutes proportions.

9. Composés selon l'une ou plusieurs des revendications 1-8, dans lesquels
R¹⁴, R¹⁵ désignent H,
et les solvats, sels et stéréoisomères pharmaceutiquement utilisables de ceux-ci, y compris des mélanges de ceux-ci en toutes proportions.

10. Composés selon l'une ou plusieurs des revendications 1-9, dans lesquels
R¹⁶, R¹⁷ désignent H,
et les solvats, sels et stéréoisomères pharmaceutiquement utilisables de ceux-ci, y compris des mélanges de ceux-ci en toutes proportions.

11. Composés selon l'une ou plusieurs des revendications 1-10, dans lesquels
R¹⁸, R¹⁹ désignent H,
et les solvats, sels et stéréoisomères pharmaceutiquement utilisables de ceux-ci, y compris des mélanges de ceux-ci en toutes proportions.

12. Composés selon l'une ou plusieurs des revendications 1-11, dans lesquels
R²⁰ désigne H,
R²¹ désigne H ou CH₂OH,
et les solvats, sels et stéréoisomères pharmaceutiquement utilisables de ceux-ci, y compris des mélanges de ceux-ci en toutes proportions.

13. Composés selon l'une ou plusieurs des revendications 1-12, dans lesquels
Hét désigne un hétérocycle mono- ou bicyclique saturé, insaturé
ou aromatique ayant de 1 à 4 atomes de N, O et/ou S, pouvant être mono- ou disubstitué par NR¹⁴R¹⁵ et/ou =O (oxygène carbonyle),
et les solvats, sels et stéréoisomères pharmaceutiquement utilisables de ceux-ci, y compris des mélanges de ceux-ci en toutes proportions.

14. Composés selon l'une ou plusieurs des revendications 1-13, dans lesquels
Hét désigne pipéridinyle, pipérazinyle, pyrrolidinyle, morpholinyle, furyle, thiényle, pyrrolyle, imidazolyle, pyrazolyle, oxazolyle, isoxazolyle, thiazolyle, isothiazolyle, pyridyle, pyrimidinyle, triazolyle, tétrazolyle, oxadiazolyle, thiadiazolyle, pyridazinyle, pyrazinyle, benzimidazolyle, benzotriazolyle, indolyle, benzo- 1,3-dioxolyle, indazolyle ou benzo-2,1,3-thiadiazolyle, chacun d'entre eux étant non substitué ou mono- ou disubstitué par NR¹⁴R¹⁵ et/ou =O (oxygène carbonyle),
et les solvats, sels et stéréoisomères pharmaceutiquement utilisables de ceux-ci, y compris des mélanges de ceux-ci en toutes proportions.

15. Composés selon l'une ou plusieurs des revendications 1-14, dans lesquels
A désigne alkyle linéaire ou ramifié ayant 1,2,3,4,5 ou 6 atomes de C, où 1-5 atomes de H peuvent être remplacés par F,
Hét désigne pipéridinyle, pipérazinyle, pyrrolidinyle, mor- pholinyle, furyle, thiényle, pyrrolyle, imidazolyle, pyrazolyle, oxazolyle, isoxazolyle, thiazolyle, isothia- zolyle, pyridyle, pyrimidinyle, triazolyle, tétrazolyle, oxadiazolyle, thiadiazolyle, pyridazinyle, pyrazinyle, benzimidazolyle, benzotriazolyle, indolyle, benzo-1,3- dioxolyle, indazolyle ou benzo-2,1,3-thiadiazolyle, chacun d'entre eux étant non substitué ou mono- ou disubstitué par NR¹⁴R¹⁵ et/ou =O (oxygène carbo- nyle),
R¹, R², R³, R⁴ désignent chacun, indépendamment les uns des autres, H, CH₃, F ou Cl,
R⁵, R⁶, R⁷, R⁸ désignent chacun, indépendamment les uns des autres, H, CH₃, Hal ou OCH₃, où R⁸ est absent si Q = N,
R⁹, R¹³ désignent chacun, indépendamment les uns des autres, CH₃, F ou Cl,
R désigne CO-NR¹⁸R¹⁹ ou Hét,
R¹¹ désigne H ou CH₃,
R¹⁰, R¹² désignent H,
Q désigne N ou C,
X désigne O,
W désigne (CR¹⁴R¹⁵)ₘ ou CO,
Z désigne (CR²⁰R²¹)ₘ,
R¹⁴, R¹⁵ désignent H,
R¹⁶, R¹⁷ désignent H,
R¹⁸, R¹⁹ désignent H,
R²⁰ désigne H,
R²¹ désigne H ou CH₂OH,
R¹⁸ et R²¹ désignent aussi ensemble (CR¹⁶R¹⁷)ₚ,
m désigne 0, 1 ou 2,
p désigne 1 ou 2,
et les solvats, sels et stéréoisomères pharmaceutiquement utilisables de ceux-ci, y compris des mélanges de ceux-ci en toutes proportions.

16. Composés selon la revendication 1, choisis parmi le groupe constitué par
| n° | Nom |
|---|---|
| "A1" | 2-{2-[1-(2,6-Diméthylbenzyl)-6-fluoro-1*H*-benzimidazol-2-yl]-5-méthoxy-3-méthylphénoxy}acétamide |
| "A2" | 2-{2-[1-(2,6-Diméthylbenzyl)-1*H*-benzimidazol-2-yl]-phénoxy}acétamide |
| "A3" | 2-{2-[1-(2,6-Diméthylbenzyl)-1*H*-benzimidazol-2-yl]-3-fluorophénoxy}acétamide |
| | 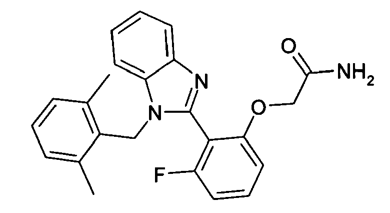 |
| "A4" | 2-{2-[1-(2,6-Diméthylbenzyl)-6-fluoro-1*H*-benzimidazol-2-yl]phénoxy}acétamide |
| | 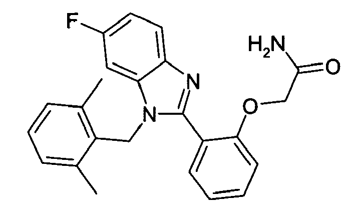 |
| "A5" | 2-{2-[1-(2,6-Diméthylbenzyl)-5,6-difluoro-1*H*-benzimidazol-2-yl]phénoxy}acétamide |
| "A6" | 2-{2-[6-Fluoro-1-(2,4,6-triméthylbenzyl)-1*H*-benzimidazol-2-yl]phénoxy}acétamide |
| "A7" | 2-{2-[1-(2-Méthylbenzyl)-1*H*-benzimidazol-2-yl]-phénoxy}acétamide |
| "A8" | 2-[2-(1-Benzyl-1*H*-benzimidazol-2-yl)phénoxy]acétamide |
| "A9" | 2-{2-[1-(2,6-Difluorobenzyl)-1*H*-benzimidazol-2-yl]-phénoxy}acétamide |
| "A10" | 2-{2-[1-(2,6-Diméthylbenzyl)-1*H*-benzimidazol-2-yl]-6-fluorophénoxy}acétamide |
| "A11" | 2-{2-[1-(2,6-Diméthylbenzyl)-1*H*-benzimidazol-2-yl]-6-méthoxyphénoxy}acétamide |
| "A12" | 2-{2-[1-(2,6-Diméthylbenzyl)-1*H*-benzimidazol-2-yl]-6-méthylphénoxy}acétamide |
| "A13" | 2-{2-[1-(2,6-Diméthylbenzyl)-1*H*-benzimidazol-2-yl]-5-méthoxyphénoxy}acétamide |
| "A14" | 2-{2-[1-(2,6-Diméthylbenzyl)-1*H*-benzimidazol-2-yl]-5-méthylphénoxy}acétamide |
| "A15" | 2-{2-[1-(2,6-Diméthylbenzyl)-1*H*-benzimidazol-2-yl]-3-méthoxyphénoxy}acétamide |
| "A16" | 2-{2-[1-(2,6-Diméthylbenzyl)-1*H*-benzimidazol-2-yl]-pyridin-3-yloxy}acétamide |
| | 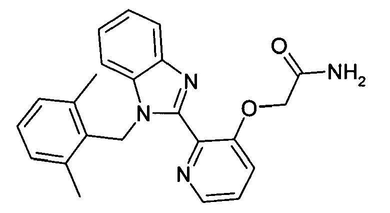 |
| "A17" | 2-{2-[1-(2,6-Diméthylbenzyl)-1*H*-benzimidazol-2-yl]-5-fluorophénoxy}acétamide |
| "A18" | 2-{2-[5-Chloro-1-(2,6-dichlorobenzyl)-1*H*-benzimidazol-2-yl]phénoxy}acétamide |
| "A19" | 2-{2-[1-(2,6-Dichlorobenzyl)-5-fluoro-1*H*-benzimidazol-2-yl]phénoxy}acétamide |
| "A20" | 2-{2-[1-(2-Chloro-6-fluorobenzyl)-1*H*-benzimidazol-2-yl]-phénoxy}acétamide |
| "A21" | 2-{2-[7-Chloro-1-(2,6-dichlorobenzyl)-1*H*-benzimidazol-2-yl]phénoxy}acétamide |
| "A22" | 2-{2-[1-(2-Chloro-6-méthylbenzyl)-1*H*-benzimidazol-2-yl]phénoxy}acétamide |
| "A23" | 2-{2-[7-Bromo-1-(2,6-diméthylbenzyl)-1*H*-benzimidazol-2-yl]phénoxy}acétamide |
| "A24" | 2-{2-[7-Chloro-1-(2,6-diméthylbenzyl)-1*H*-benzimidazol-2-yl]phénoxy}acétamide |
| "A25" | 2-{2-[1-(2,6-Diméthylbenzoyl)-1*H*-benzimidazol-2-yl]-phénoxy}acétamide |
| | 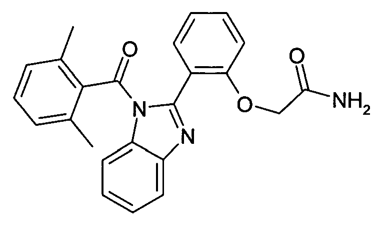 |
| "A26" | 3-{2-[1-(2,6-Diméthylbenzyl)-1*H*-benzimidazol-2-yl]phénoxy}pyrrolidin-2-one |
| | 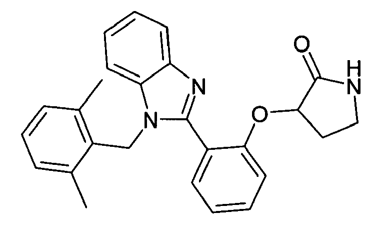 |
| "A27" | 2-{2-[1-(2,6-Diméthylbenzyl)-1*H*-benzimidazol-2-yl]-phénoxy}-3-hydroxypropionamide |
| | 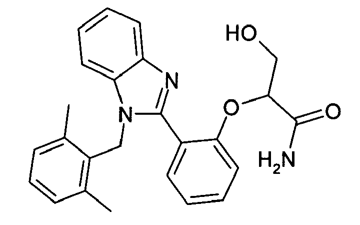 |
| "A28" | 2-{2-[1-(2,6-Diméthylbenzyl)-5,6-difluoro-1*H*-benzimidazol-2-yl]-5-méthoxy-3-méthylphénoxy}acétamide |
| "A29" | 2-{2-[1-(2,6-Diméthylbenzyl)-6,7-difluoro-1*H*-benzimidazol-2-yl]phénoxy}acétamide |
| "A30" | 2-{2-[1-(2,6-Diméthylbenzyl)-6,7-difluoro-1*H*-benzimidazol-2-yl]-5-méthoxy-3-méthylphénoxy}acétamide |
et les solvats, sels et stéréoisomères pharmaceutiquement utilisables de ceux-ci, y compris des mélanges de ceux-ci en toutes proportions.

17. Procédé de préparation de composés de formule I selon les revendications 1-16 et de solvats, sels et stéréoisomères pharmaceutiquement utilisables de ceux-ci, **caractérisé en ce que**
a) un composé de formule II 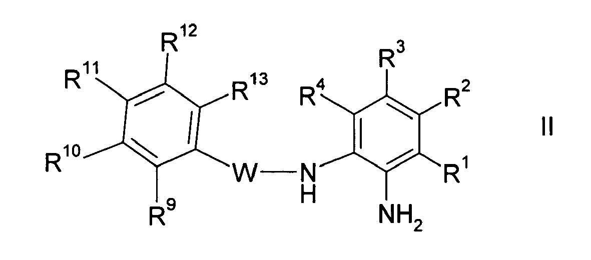 dans laquelle
R¹, R², R³, R⁴, R⁹, R¹⁰, R¹¹, R¹², R¹³ et W revêtent les significations indiquées selon la revendication 1,
est réagi avec un composé de formule III 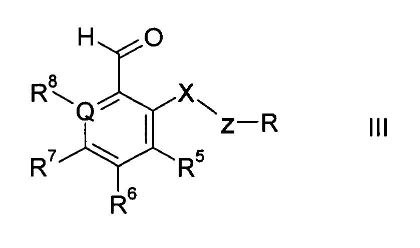 dans laquelle
R⁵, R⁶, R⁷, R⁸, R, Q, X et Z revêtent les significations indiquées selon la revendication 1,
ou
b) un composé de formule IV 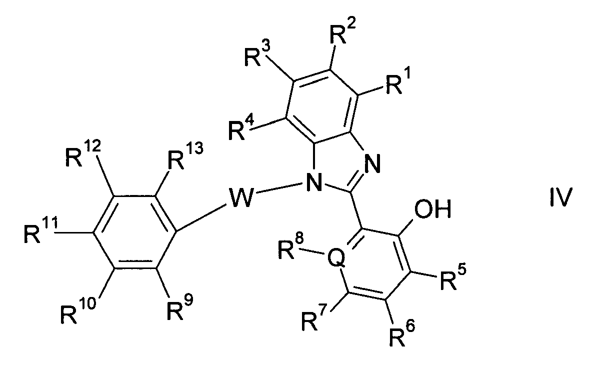 dans laquelle
R¹, R², R³, R⁴, R⁵, R⁶, R⁷, R⁸, R⁹, R¹⁰, R¹¹, R¹², R¹³, Q et W revêtent les significations indiquées selon la revendication 1,
est réagi avec un composé de formule V
L-Z-R V
dans laquelle Z et R revêtent les significations indiquées selon la revendication 1, et
L désigne Cl, Br, I ou un groupement OH libre ou modifié de manière réactive et fonctionnelle,
et/ou
une base ou un acide de formule I est converti(e) en l'un de ses sels.

18. Médicaments comprenant au moins un composé de formule I selon l'une ou plusieurs des revendications 1 à 16 et/ou des solvats, sels et stéréoisomères pharmaceutiquement utilisables de celui-ci, y compris des mélanges de ceux-ci en toutes proportions, et éventuellement des excipients et/ou adjuvants.

19. Médicaments comprenant au moins un composé de formule I selon l'une ou plusieurs des revendications 1 à 16 et/ou de solvats et stéréoisomères pharmaceutiquement utilisables de celui-ci, y compris des mélanges de ceux-ci en toutes proportions, et au moins un autre ingrédient actif médicamenteux.

20. Utilisation de composés selon l'une ou plusieurs des revendications 1 à 16 et/ou de sels et solvats physiologiquement acceptables de ceux-ci pour la préparation d'un médicament destiné au traitement du diabète de type 1 et de type 2.

21. Utilisation de composés selon l'une ou plusieurs des revendications 1 à 16 et/ou de sels et solvats physiologiquement acceptables de ceux-ci pour la préparation d'un médicament destiné à abaisser le taux de sucres dans le sang.

22. Utilisation de composés selon l'une ou plusieurs des revendications 1 à 16 et/ou de sels et solvats physiologiquement acceptables de ceux-ci et d'un autre ingrédient actif médicamenteux pour la préparation d'un médicament destiné au traitement du diabète de type 1 et de type 2.

23. Ensemble (kit) constitué de conditionnements séparés
(a) d'une quantité efficace d'un composé de formule I selon l'une ou plusieurs des revendications 1 à 16 et/ou de solvats, sels et stéréoisomères pharmaceutiquement utilisables de celui-ci, y compris des mélanges de ceux-ci en toutes proportions,
et
(b) d'une quantité efficace d'un autre ingrédient actif médicamenteux.

24. Utilisation de composés selon l'une ou plusieurs des revendications 1 à 16 et/ou de sels et solvats physiologiquement acceptables de ceux-ci et d'un autre ingrédient actif médicamenteux pour la préparation d'un médicament destiné à abaisser le taux de sucres dans le sang.

25. (5-{2-[1-(2,6-Diméthylbenzyl)-1*H*-benzimidazol-2-yl]phényl}-1,3,4-oxadiazol-2-yl)méthylamine, et sels pharmaceutiquement utilisables de celle-ci.
